(19) Europäisches Patentamt
European Patent Office
Office européen des brevets

(11) EP 4 775 566 A1

(12) **EUROPEAN PATENT APPLICATION**

(43) Date of publication:
**15.07.2026 Bulletin 2026/29**

(21) Application number: **25151378.4**

(22) Date of filing: **10.01.2025**

(51) International Patent Classification (IPC):
***C07C 217/16*** *(2006.01)* ***C07C 219/14*** *(2006.01)*
***C07D 295/088*** *(2006.01)* ***A61K 9/1272*** *(2025.01)*

(52) Cooperative Patent Classification (CPC):
**C07C 217/16; A61K 9/5123; C07C 219/14; C07D 295/088; C07D 317/46**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **SYVENTO SP. Z O.O.**
**32-050 Skawina (PL)**

(72) Inventors:
- **LIPKA, Dominik**
  **32-392 Kraków (PL)**
- **ZAWILSKA, Patrycja**
  **58-200 Dzierzoniów (PL)**
- **DLUGOSZ-GROCHOWSKA, Olga**
  **30-348 Kraków (PL)**
- **FURMAN, Bartlomiej**
  **05-126 Wólka Radzyminska (PL)**
- **GRZESZCZYK, Barbara**
  **02/793 Warszawa (PL)**

(74) Representative: **Eisenführ Speiser**
**Patentanwälte Rechtsanwälte PartGmbB**
**Gollierstraße 4**
**80339 München (DE)**

(54) **COMPOUNDS FOR ENCAPSULATION AND DELIVERY**

(57) The present invention relates to a novel compound, an ionizable lipid, an ionizable lipid nanoparticle, a method of producing such an ionizable lipid, a method of producing such an ionizable lipid nanoparticle, an ionizable lipid and an ionizable lipid nanoparticle obtainable by such a method, a composition comprising said compound or ionizable lipid or ionizable lipid nanoparticle, as well as the use of such a compound, ionizable lipid, ionizable lipid nanoparticle or composition for medical treatment and/or prevention and for further areas of application.

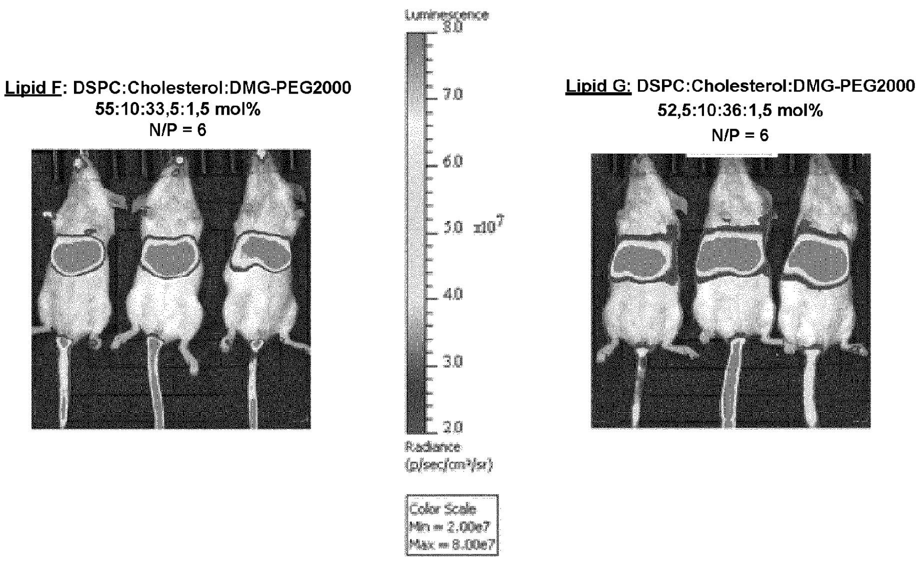


Fig. 1

EP 4 775 566 A1

## Description

### Technical Field

The present invention relates to a novel compound, an ionizable lipid, an ionizable lipid nanoparticle, a method of producing such an ionizable lipid, a method of producing such an ionizable lipid nanoparticle, an ionizable lipid and an ionizable lipid nanoparticle obtainable by such a method, a composition comprising said compound or ionizable lipid or ionizable lipid nanoparticle, as well as the use of such a compound, ionizable lipid, ionizable lipid nanoparticle or composition for medical treatment and/or prevention and for further areas of application.

### Background of the invention

Delivering an active ingredient to a specific target site has been of great importance in research for decades. To provide the best effect possible it was found to be mandatory the active ingredient to be part of a formulation that manages to get exactly to the place where it is needed to be released, absorbed or distributed and to act specifically. On this way the active ingredient needs to be protected from possible destruction. At best, high bioavailability and low toxicity to the target organism should be ensured. Therefore, amphiphilic molecules were deemed to be effective delivery systems for molecules. More than just serving as delivery system they can also be used as main ingredient to target specific areas or receptors. Many small molecules are easily destructible, if not protected, but should nevertheless be transported to a specific location to take effect there. It was found that nanoparticles have useful properties for this purpose. They can increase the bioavailability of such molecules, transport substances specifically to their site of action and - with the aid of certain surfactants - even cross the blood-brain barrier for specific drug targeting.

Having both, lipophilic and hydrophilic parts, also liposomes and microemulsions are often used as delivery system. Therefore, appropriate compounds such as lipids are useful. Nanocrystals, nanospheres and nanocapsules are more examples of molecule delivery systems. Containing lipid structures combined with the characteristics of nanoparticles, makes lipid nanoparticle (LNP) technology gaining significant importance. Due to the insight to be among the most effective molecule delivery systems it is especially used in the field of nucleic acid delivery, such as mRNA, siRNA, linear DNA, antisense nucleotide, oligonucleotide, aptamer, miRNA and pDNA, for gene therapy, e.g. in combination with the CRISPR-Cas9 method, and mRNA vaccines. Also in fields like cosmetics, nutrition, the agricultural sector, protein replacement, self-replicating, CAR-T cell therapy, in-vivo cell therapy, immunotherapy and cancer therapy an increasing use is possible.

As a special form of lipid nanoparticles prove to be ionizable lipid nanoparticles and the ionizable lipids contained therein. These lipids, due to their ability to change charge depending on the pH, enable effective encapsulation, protection, and delivery of small molecules like nucleic acids to target locations, such as cells, for example.

Contemporary LNP systems rely on ionizable lipids with specific chemical structures that can acquire a positive charge in acidic environments, which is crucial for their function. At neutral or basic pH, these lipids are neutral, minimizing their toxicity, while in acidic environments, such as endosomes, they ionize. That means at low pH the positive charges of the lipid ensure binding to a negatively charged basis. E.g. in case of binding to the negatively charged mRNA, the positively charged ionizable lipids promote endosomal membrane destabilization and the release of the payload into the cytoplasm. Along with further lipids, the ionizable lipids form a protective envelope around the sensitive molecules, such as nucleic acids, and thus ensure that they can be safely and efficiently introduced into target locations.

A famous application of such LNP technology is the ionizable lipid *D-Lin-MC3-DMA* which is used in Patisiran, commercially used as *Onpattro®*. Patisiran is a drug for the treatment of hereditary ATTR amyloidosis (hATTR), based on RNA interference, a so-called siRNA. That means, siRNA has been encapsulated in a LNP to be protected and delivered to a special target location, in this case a mRNA region of the target organism coding for a protein called transthyretin.

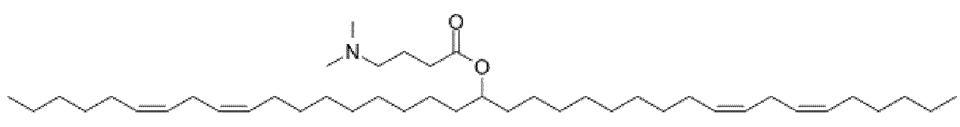

*D-Lin-MC3-DMA*

*SM-102* by *Moderna®* and *ALC-0315* by *BioNTech®/Pfizer®* are further prominent examples for ionizable lipids and their use in LNP technology. They have been used in formulations for mRNA based vaccines against Covid-19.

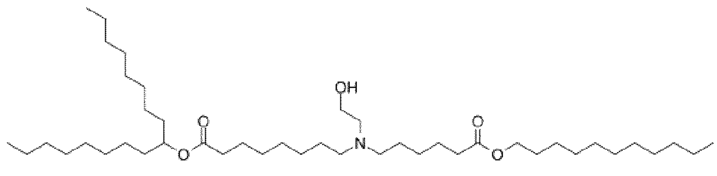

*SM-102*

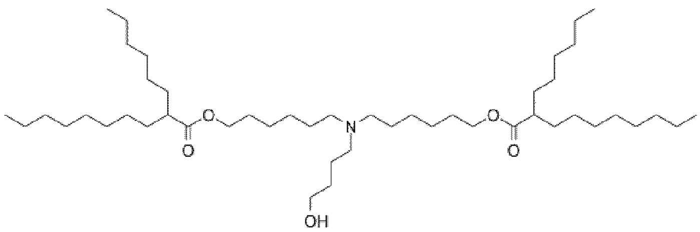

*ALC-0315*

*D-Lin-MC3-DMA, SM-102* and *ALC-0315* all disclose a chemical structure comprising an aliphatic tertiary amine as the ionizable part. Along with one or two ester groups there are disclosed aliphatic hydrocarbon chains. All of these structures do not disclose any aromatic structure at all. While *D-Lin-MC3-DMA, SM-102* and *ALC-0315* are all used to encapsulate and deliver a specific siRNA or mRNA and are made exclusively for one specific medical treatment or in one pharmaceutical composition, respectively, literature and current practice are completely silent about further formulations and uses.

US 11,766,408 B2 discloses ionizable lipids and SORT (selecting organ targeting) lipids that are permanently protonated as well as compositions comprising them. Apart from D-*Lin-MC3-DMA* the ionizable lipids disclosed may comprise cyclic hydrocarbon compounds, mainly heteroaryl or heterocycloalkyl groups, while they hardly comprise benzoic structures. Most lipid chains connected to an ester group disclosed in said patent are exactly connected to the carbon atom of the ester group. According to the patent, the ionizable lipids can be used to deliver a composition for delivery of a therapeutic agent such as a nucleic acid, a protein or a small molecule to a particular organ. An alternative target organism or further payload let alone other uses are not mentioned.

WO 2021/055833 A1 discloses specific compounds, compositions, empty and loaded LNPs and methods, and describes branched tail lipid compounds and compositions for intracellular delivery therapeutic agents. The chemical structures have mainly aliphatic lipids, while a possible cyclic and aromatic group is directly connected to the oxygen atom of an ester group and the carbon chain between the aliphatic amine and the ester group is directly connected to the carbon atom of the ester group. The document does not mention any further use than the medical or therapeutic one.

The article of Tang et al., "Ionizable Lipid Nanoparticles for mRNA Delivery", 2023, concentrates on mRNA delivery in ionizable LNPs and discloses several ionizable lipids. Among them there is *D-Lin-MC3-DMA.* Beside other aliphatic lipid groups there are also shown aromatic groups, wherein a benzene can be connected to an ester group either directly to an oxygen atom or via a methylene group. In this special example, the ionizable lipid called *ssPalmO-Phe* also has a disulfide bridge and the tertiary amine is part of a heterocycle. All disclosed ionizable LNPs and the ionizable lipids used therein are exclusively used for mRNA delivery in a therapeutical field, the article is completely silent about further uses.

Considering the advantageous effects and immense possibilities of the LNP technology in general and the compounds used therein, there is a continuous need to search for new ionizable lipid structures and even completely new chemical structural classes of lipids suitable as ionizable lipids. The goal is to improve the efficiency of molecule delivery to specific locations in versatile target organisms, whether it is the human body with its usual cell structure, skin barrier and even beyond the blood-brain barrier. Also other organisms than the human body can be target organisms like, e.g., plant cells due to the great capability of compounds suitable as ionizable lipids and whole ionizable LNPs passing through biomembranes. Therefore, there is need for new ionizable lipids and LNP formulations in further application areas than just immune modulation or drug targeting in the therapeutic field, such as cosmetics, nutrition and the agricultural sector. Besides, it is required by new compounds suitable as ionizable lipids as both, autonomous main ingredient and preferably as carrier lipid, to have a high bioavailability and to be easily tissue compatible. There is further a need of compounds suitable as ionizable lipids being able to reduce toxicity for the target organism and to increase the stability of LNP formulations with the aim of protecting the main ingredient and the formulation itself. Combining these desirable properties in one single lipid could lead to be able to use stable and highly functional compounds, LNPs or compositions for an immensely wide range of applications.

## Summary of the invention

The present invention solves the above needs by providing, in a first aspect, a compound being represented by formula (A) or formula (B) or a salt or isomer thereof, preferably being suitable as lipid, particularly preferably being used as ionizable lipid

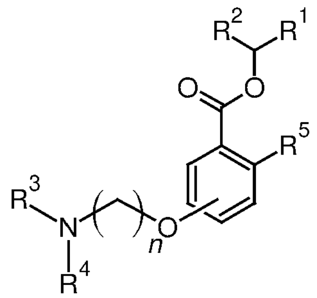

formula (A)

wherein $R_1$ is an alkyl, an alkenyl or an alkinyl,

wherein $R_2$ is selected from the group consisting of H, COOH, benzyl, phenol, an alkyl, and an alkenyl,

wherein $R_3$ and $R_4$ are independently selected from the group consisting of H, $NH_2$, benzyl, phenol, benzoic acid, an alkyl, and an alkenyl,

wherein $R_5$ is selected from the group consisting of H, $NH_2$, an alkyl, an alkenyl or an alkinyl,

wherein n is 1, 2, 3, 4, 5 or 6, preferably 1, 2, 3 or 4, more preferably 2 or 3, particularly preferably 2,

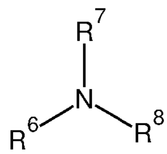

formula (B),

wherein $R_6$ is represented by formula (B-1)

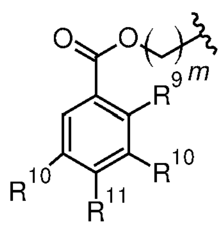

formula (B-1),

or a salt or isomer thereof,

wherein $R_7$ is represented by formula (B-1) or a salt or isomer thereof, or is selected from the group consisting of H, an alkyl with a total number of carbon atoms in the range from 1 to 7, a methanol group, an ethanol group, a propanol group, a butanol group or a pentanol group, preferably a methyl group,

wherein $R_8$ is represented by formula (B-1) or a salt or isomer thereof, or is selected from the group consisting of H, an alkyl with a total number of carbon atoms in the range from 1 to 7, a methanol group, an ethanol group, a propanol group, a butanol group or a pentanol group, preferably a methyl group or a propanol group,

wherein $R_9$ is selected from the group consisting of H, O-$R_{B1}$ or an alkyl,

wherein $R_{B1}$ is selected from the group consisting of H, OH or an alkyl,

wherein $R_{10}$ is selected from the group consisting of H, O-$R_{B2}$ or an alkyl,

wherein $R_{B2}$ is selected from the group consisting of H, OH or an alkyl,

wherein $R_{11}$ is selected from the group consisting of H, O-$R_{B3}$ or an alkyl,

wherein $R_{B3}$ is selected from the group consisting of H, OH or an alkyl, and/or

wherein m is 1, 2, 3, 4, 5 or 6, preferably 2, 3, 4 or 5, particularly preferably 2 or 5,

preferably wherein at least one of $R_9$, $R_{10}$ or $R_{11}$ is selected from the group consisting of $OR_{B1}$, O-$R_{B2}$ or O-$R_{B3}$.

In one embodiment, there is provided a compound according to the first aspect, wherein the at least one tertiary amine is selected from the group of a tertiary aliphatic amine, a tertiary cyclic amine and a tertiary aromatic amine, preferably wherein the at least one tertiary amine is a tertiary aliphatic amine.

In another embodiment, there is provided a compound according to the first aspect, wherein further at least one of $R_1$, $R_2$, $R_5$, $R_9$, $R_{10}$, $R_{11}$, $R_{B1}$, $R_{B2}$ and $R_{B3}$ is/are an alkyl, an alkenyl or an alkinyl with a total number of carbon atoms of 1, 2, 3, 4, 5, 6, 7, 8, 9, 10, 11, 12, 13, 14, 15, 16, 17, 18, 19, 20, 21, 22, 23, 24, 25, or above, preferably with a total number of carbon atoms in the range from 6 to 24, more preferably wherein at least one of $R_1$, $R_2$, $R_5$, $R_9$, $R_{10}$, $R_{11}$, $R_{B1}$, $R_{B2}$ and $R_{B3}$ has a total number of carbon atoms of 10, 12, 13, 15, 16, 17 or 18.

In yet another embodiment, there is provided a compound according to the first aspect, wherein further the alkyl or alkenyl or alkinyl comprises one or more hetero atoms as chemical residue, preferably wherein the hetero atom(s) is/are oxygen atom(s).

In a further embodiment, there is provided a compound according to the first aspect being represented by formula (1), (2), (3), (4), (5), (6), (7), (8), (9), (10), (11), (12), (13) or (14) or a salt or isomer thereof, preferably being suitable as lipid, particularly preferably being used as ionizable lipid

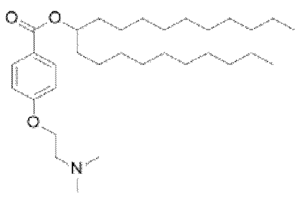

formula (1)

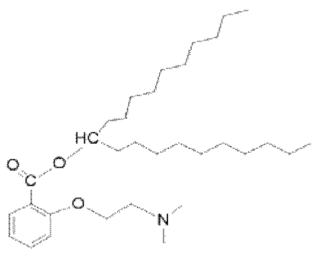

formula (2)

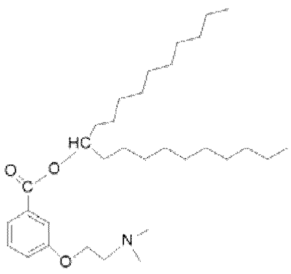

formula (3)

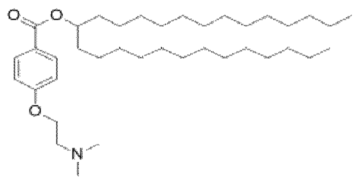

formula (4)

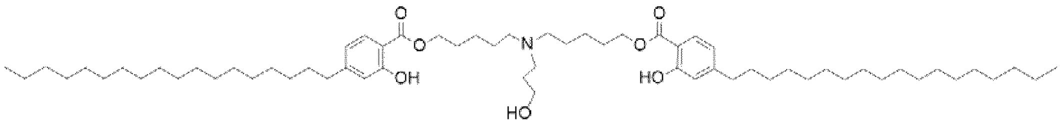

formula (5)

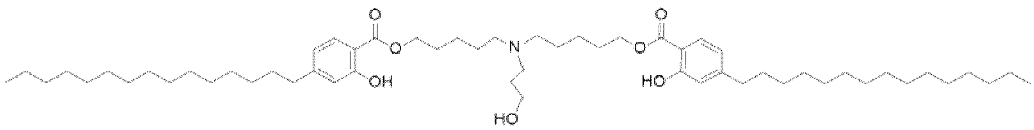

formula (6)

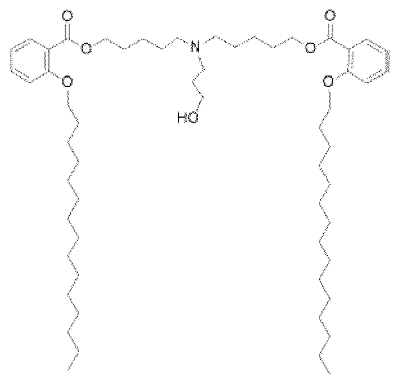

formula (7)

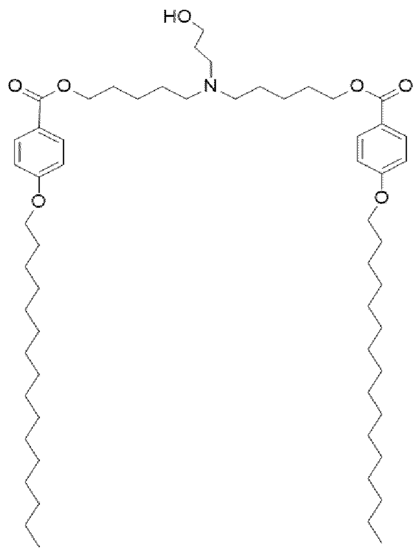

formula (8)

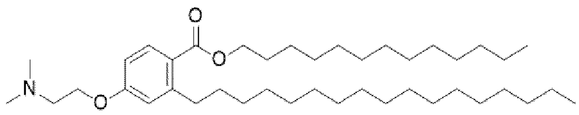

formula (9)

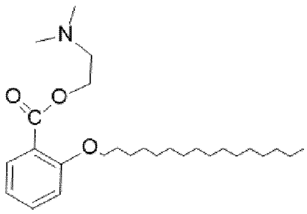

formula (10)

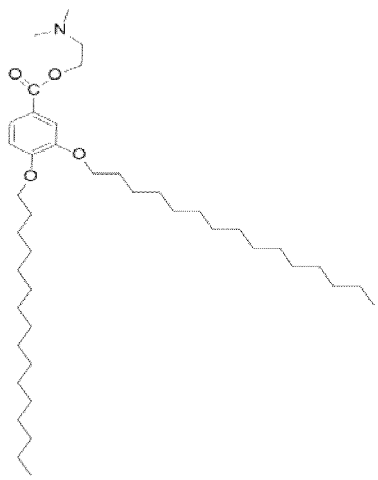

formula (11)

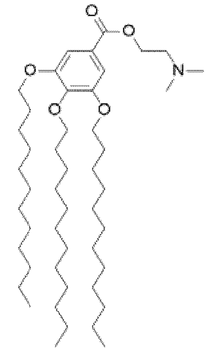

formula (12)

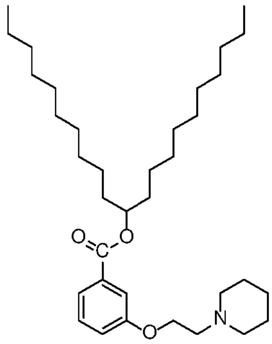

formula (13)

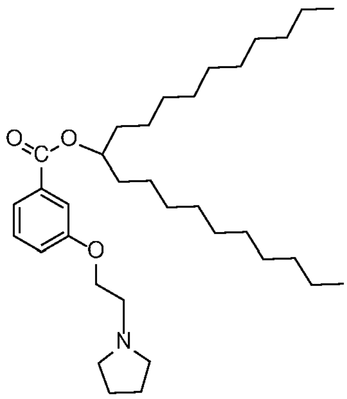

formula (14).

In a second aspect, there is provided an ionizable lipid according to the first aspect and the various embodiments related thereto, comprising a hydroxybenzoic acid structure,

preferably wherein the ionizable lipid has a chemical structure of formula (A) or (B), wherein formula (B) includes formula (B-1), as defined above,

preferably wherein one or two hydroxybenzoic acid(s) is/are present.

In one embodiment, there is provided an ionizable lipid according to the second aspect, wherein at least one tertiary amine is present and wherein the at least one tertiary amine has at least one chemical residue, wherein the chemical residue is selected from the group consisting of H, an alkyl, a methanol group, an ethanol group, a propanol

group, a butanol group or a pentanol group, preferably a methyl group or a propanol group,

preferably wherein the tertiary amine is connected to the phenolic oxygen atom of the hydroxybenzoic acid via an alkyl group,

preferably wherein the tertiary amine is connected to the oxygen atom of the carbonyl group of the hydroxybenzoic acid via an alkyl group,

preferably wherein the carboxyl group of the hydroxybenzoic acid is connected to the tertiary amine via an alkyl group,

preferably wherein the aromatic group of the hydroxybenzoic acid has H, an ether or an alkyl as a chemical residue,

preferably wherein the tail group as chemical residue of the ether and/or the aromatic group of the hydroxybenzoic acid and/or the oxygen atom of the carboxyl group is an alkyl, an alkenyl or an alkinyl with a total number of carbon atoms of 1, 2, 3, 4, 5, 6, 7, 8, 9, 10, 11, 12, 13, 14, 15, 16, 17, 18, 19, 20, 21, 22, 23, 24, 25, or above, more preferably with a total number of carbon atoms in the range from 6 to 24, particularly preferably with a total number of carbon atoms of 10, 12, 13, 15, 16, 17 or 18.

In a third aspect, there is provided a method of producing the compound according to the first aspect and the various embodiments related thereto, or the lipid according to the second aspect and the embodiments related thereto,

wherein the method comprises the following steps, in sequence:

a) providing a raw material comprising a structure of hydroxbenzoic acid or a derivative or a salt thereof,

b) mixing the provided raw material with a substance for elongating the tail group of the provided raw material,

c) adding a substance to the mixture obtained in b) for hydrating a derivative of a hydroxyl group of the mixture obtained in b),

d) adding a substance comprising a tertiary amine to the mixture obtained in c), and

e) obtaining a product comprising a hydroxybenzoic acid structure connected to a tertiary amine and further comprising a lipophilic tail group.

In a fourth aspect, there is provided an ionizable lipid nanoparticle comprising the compound according to the first aspect and the various embodiments related thereto or the lipid according to the second aspect and the embodiments related thereto.

In one embodiment, there is provided an ionizable lipid nanoparticle according to the fourth aspect, further comprising

structure lipids, wherein the structure lipid is phospholipid and/or sphingolipid, preferably wherein the phospholipid is selected from the group consisting of PC, PE, PA, PG, PI, PS and LPL, preferably wherein the sphingolipid is sphingomyelin, more preferably wherein the phospholipid is selected from the group of DSPC, DOPE, HSPC, DPPC, DSPG, DSPS and DPPA,

sterols, preferably wherein the sterol is cholesterol and/or sitosterol,

lipids modified with hydrophilic polymers, preferably wherein the lipid modified with hydrophilic polymers is PEGylated lipid, more preferably wherein the PEGylated lipid is DMG-PEG and/or DSPE-PEG and/or DSG-PEG,

and optionally

SORT lipids, preferably wherein the SORT lipid comprises DODAP, DOTAP, DPDAB, DODMA, 14PA, 18BMP, 18PA, DC-cholesterol and/or 5A2-SC8,

and optionally

antioxidants and/or oils and/or fatty acids and/or fatty acid salts and/or surfactants.

In a fifth aspect, there is provided a method of producing the ionizable lipid nanoparticles according to the fourth aspect and the embodiments related thereto,
wherein the method comprises the following steps, in sequence:

a) providing a lipid phase comprising a compound according to any of claims 1 to 5, an ionizable lipid according to claim 6 or 7 or a product obtained according to claim 8,

b) providing an aqueous phase,

c) mixing the lipid phase provided in a) with the aqueous phase provided in b), preferably wherein a microfluidic technique is used, and

d) obtaining a lipid nanoparticle.

In a sixth aspect, there is provided a composition comprising the compound according to the first aspect and the various embodiments related thereto or the ionizable lipid according to the second aspect and the embodiments related thereto or the ionizable lipid nanoparticle according to the fourth aspect and the embodiments related thereto,
preferably wherein the composition is a pharmaceutical composition, a cosmetic composition, a nutritional composition or an agricultural composition.

In a seventh aspect, there is provided a compound according to the first aspect, or an ionizable lipid according to the second aspect, or an ionizable lipid nanoparticle according to fourth aspect, or a composition according to the sixth aspect, for use in a method of treatment and/or prevention as an active ingredient or as a cargo of an active ingredient.

In an eighth aspect, there is provided a compound according to the first aspect, or an ionizable lipid according to the second aspect, or an ionizable lipid nanoparticle according to fourth aspect, or a composition according to the sixth aspect, for use in a method of treatment and/or prevention as an active ingredient or as a cargo of an active ingredient, wherein at least one disease selected from the group of cancer, inflammatory diseases, autoimmune diseases, destroyed tissue, psychological disorders, gastrointestinal diseases and respiratory diseases is/are treated and/or prevented.

In a variant aspect according to the seventh aspect, there is provided a use of the compound according to the first aspect and the various embodiments related thereto or a use of the ionizable lipid according to the second aspect and the embodiments related thereto or a use of the ionizable lipid nanoparticle according to the fourth aspect and the embodiments related thereto or a use of the composition according to the sixth aspect for the manufacture or preparation of a medicament for treatment and/or prevention of a disease or health condition as an active ingredient or as a cargo of an active ingredient.

In a variant aspect according to the eighth aspect, there is provided a use of the compound according to the first aspect and the various embodiments related thereto or a use of the ionizable lipid according to the second aspect and the embodiments related thereto or a use of the ionizable lipid nanoparticle according to the fourth aspect and the embodiments related thereto or a use of the composition according to the sixth aspect for the manufacture or preparation of a medicament for treatment and/or prevention of a disease or health condition as an active ingredient or as a cargo of an active ingredient,
wherein at least one disease selected from the group of cancer, inflammatory diseases, autoimmune diseases, destroyed tissue, psychological disorders, gastrointestinal diseases and respiratory diseases is/are treated and/or prevented.

In a variant aspect according to the seventh and eighth aspect there is provided a method of treatment or prevention of a disease or health condition, comprising administering a compound or an ionizable lipid or an ionizable lipid nanoparticle or a composition according to the various aspects to a patient in need thereof. Optionally, said method comprises administering a pharmaceutically acceptable carrier or excipient.

In one embodiment the disease to be treated or prevented is cancer. In another embodiment the disease to be treated or prevented is an inflammatory disease. In yet another embodiment the disease to be treated or prevented is an autoimmune disease. In a further embodiment the health condition to be treated or prevented is destroyed tissue. In yet a further embodiment the disease to be treated or prevented is a psychological disorder. In still another embodiment the disease to be treated or prevented is a gastrointestinal disease. In an even further embodiment the disease to be treated or prevented is a respiratory disease.

In a ninth aspect, there is provided a use of the compound according to the first aspect and the various embodiments related thereto or the ionizable lipid according to the second aspect and the embodiments related thereto or the ionizable lipid nanoparticle according to the fourth aspect and the embodiments related thereto or the composition according to the sixth aspect as a cargo in a cosmetic product, in a nutritional product, in a diagnostic product, for recombinant protein production, or in an agricultural product.

## Brief description of drawings

The term "Lipid G" or "SYV-G" as used herein and specifically in the figures refers to formula (1) according to the invention. The term "Lipid O" or "SYV-O" or "LO" as used herein and specifically in the figures refers to formula (2) according to the invention. The term "Lipid P" or "SYV-P" or "LP" as used herein and specifically in the figures refers to formula (3) according to the invention. The term "Lipid S" or "SYV-S" as used herein and specifically in the figures refers to formula (4) according to the invention. The term "Lipid C" or "SYV-C" as used herein and specifically in the figures refers to formula (5) according to the invention. The term "Lipid E" or "SYV-E" as used herein and specifically in the figures refers to formula (6) according to the invention. The term "Lipid F" or "SYV-F" or "LF" as used herein and specifically in the figures refers to formula (7) according to the invention. The term "Lipid H" or "SYV-H" as used herein and specifically in the figures refers to formula (8) according to the invention. The term "Lipid K" or "SYV-K" as used herein and specifically in the figures refers to formula (9). The term "Lipid M" or "SYV-M" as used herein and specifically in the figures refers to formula (10) according to the invention. The term "Lipid N" or "SYV-N" as used herein and specifically in the figures refers to formula (11) according to the invention. The term "Lipid R" or "SYV-R" as used herein and specifically in the figures refers to formula (12) according to the invention. The term "Lipid U" or "SYV-U" as used herein and specifically in the figures refers to formula (13) according to the invention. The term "Lipid W" or "SYV-W" as used herein and specifically in the figures refers to formula (14) according to the invention. The term "Lipid T" or "SYV-T" as used herein and specifically in the figures refers to formula (15) according to the invention.

**Figure 1 (Fig. 1)** shows the in-vivo distribution of mRNA-LNPs via bioluminescence imaging. 6-8 week-old female BALB/c mice were administered dispersion of provided test formulations. As a result the accumulation and decay of bioluminescence associated with the administered mRNA-LNP formulations can be seen with the help of luciferin. The representative IVIS images show groups of female BALB/c mice injected with 5 μg Luc mRNA encapsulated by LNP formulations, comprising Lipid F and Lipid G, by the intravenous route after 24h after administration.

**Figure 2 (Fig. 2)** shows the in-vivo distribution of mRNA-LMPs via bioluminescence imaging. 6-8 week-old female BALB/c mice were administered dispersion of provided test formulations. As a result the accumulation and decay of bioluminescence associated with the administered mRNA-LNP formulations can be seen with the help of luciferin. The representative IVIS images show groups of female BALB/c mice injected with 5 μg Luc mRNA encapsulated by LNP formulations, comprising Lipid O and Lipid P, by the intravenous route after 24h after administration.

**Figure 3 (Fig. 3)** shows the bioluminescent signal shown in Fig. 1 and Fig. 2 quantified as total flux (p/s). For a better understanding the signals are compared to a group of mice that were administered LNP formulations comprising the ionizable lipid *SM-102.*

**Figure 4 (Fig. 4)** shows IC50 (half maximal Inhibitory Concentration) values of ionizable lipids according to the invention plus, for better illustration, an IC50 value of ionizable lipid *SM-102* in comparison. The higher the value, the lower is the cytotoxicity of the ionizable lipid.

**Figure 5 (Fig. 5)** shows a bar plot representing a Jurkat luminescence readout [RLU] after 24 hours of treatment. There are shown formulations according to the invention and lipofectamine transfection in an amount of 50 ng, 20 ng and 10 ng of mRNA per well, respectively. Lipofectamine transfection showed such a low luminescence, that the column is almost not visable here. For a better understanding the values of Lipid P, Lipid O and Lipid G are compared to formulations comprising the ionizable lipid *SM-102.* Error bars represent SD values.

**Figure 6 (Fig. 6)** shows a bar plot representing a Jurkat luminescence readout [RLU] divided by non-transfected cells luminescence readout after 24 hours of treatment. There are shown formulations according to the invention and lipofectamine transfection in an amount of 50ng mRNA per well. For a better understanding the values values of Lipid P, Lipid O and Lipid G are compared to formulations comprising the ionizable lipid *SM-102.* Error bars represent SD values.

**Figure 7 (Fig. 7)** shows a bar plot representing the ratio between formulations according to the invention, namely Lipid P, Lipid O and Lipid G, compared to *SM-102* in an amount of 50ng mRNA per well, respectively.

**Figure 8 (Fig. 8, A** and **B)** shows the efficiency of siRNA transfection in LNPs according to the invention, more specifically expression of Target mRNA (TNC) for siRNA-LNP in U-87 MG (Glioma) Cells Compared to Empty LNPs. Incubation for 48 Hours in the Presence of 5% FBS. The lower the value, the better. The expression values comprising LNPs according to the invention as a cargo for nucleic acids are compared to control values of LNPs that do not have

an active ingredient. The decimal numbers written on the x-axis represent the concentration of siRNA in the respective LNP in μg/mL for a comparison of target silencing levels in different concentrations. The target is Tenascin-C (TNC). 8A shows LNPs comprising lipid MC3. 8B shows LNPs comprising lipid C12-200. The expression of target mRNA (TNC) for siRNA-LNP in U-87 MG (Glioma) cells are compared to empty LNPs that comprise the same lipid composition, but do not have a payload, as control LNPs. E.g. SYV1 and SYV6 comprise the same lipid composition and were prepared at the same lipid concentration, but differ in having a payload or not. The incubation was carried out for 48 hours in Presence of 5% FBS. The lower the value, the better is the target silencing effectiveness. The level of gene expression silencing was calculated relative to the corresponding control LNP.

**Figure 9 (Fig. 9, A** and **B)** shows the efficiency of siRNA transfection in LNPs according to the invention, more specifically expression of Target mRNA (TNC) for siRNA-LNP in U-87 MG (Glioma) Cells Compared to Empty LNPs. Incubation for 48 Hours in the Presence of 5% FBS. The lower the value, the better. The expression values comprising LNPs according to the invention as a cargo for nucleic acids are compared to control values of LNPs that do not have an active ingredient. The decimal numbers written on the x-axis represent the concentration of siRNA in the respective LNP in μg/mL for a comparison of target silencing levels in different concentrations. The target is TNC. 9A shows LNPs comprising Lipid F. 9B shows LNPs comprising Lipid O. The expression of target mRNA (TNC) for siRNA-LNP in U-87 MG (Glioma) cells are compared to empty LNPs that comprise the same lipid composition, but do not have a payload, as control LNPs. E.g. SYV3 and SYV8 comprise the same lipid composition and were prepared at the same lipid concentration, but differ in having a payload or not. The incubation was carried out for 48 hours in Presence of 5% FBS. The lower the value, the better is the target silencing effectiveness. The level of gene expression silencing was calculated relative to the corresponding control LNP.

**Figure 10 (Fig. 10)** shows the efficiency of siRNA transfection in LNPs according to the invention, more specifically expression of Target mRNA (TNC) for siRNA-LNP in U-87 MG (Glioma) Cells Compared to Empty LNPs. Incubation for 48 Hours in the Presence of 5% FBS. The lower the value, the better. The expression values comprising LNPs according to the invention as a cargo for nucleic acids are compared to control values of LNPs that do not have an active ingredient. The decimal numbers written on the x-axis represent the concentration of siRNA in the respective LNP in μg/mL for a comparison of target silencing levels in different concentrations. The target is TNC. 10A shows LNPs comprising Lipid F. 10B shows LNPs comprising Lipid P. The expression of target mRNA (TNC) for siRNA-LNP in U-87 MG (Glioma) cells are compared to empty LNPs that comprise the same lipid composition, but do not have a payload, as control LNPs. E.g. SYV2 and SYV7 comprise the same lipid composition and were prepared at the same lipid concentration, but differ in having a payload or not. The incubation was carried out for 48 hours in the presence of 5% FBS. The lower the value, the better is the target silencing effectiveness. The level of gene expression silencing was calculated relative to the corresponding control LNP.

**Figure 11 (Fig. 11, A** to **F)** shows the expression analysis of immune response-related factors. The expression values comprising LNPs according to the invention as a cargo for nucleic acids are compared to control values of LNPs that do not have an active ingredient. The decimal numbers written on the x-axis represent the concentration of siRNA in the respective LNP in μg/mL for a comparison of gene expression levels in different concentrations. On the top row (11A to 11 C) there are the values of oligoadenylate synthetase 1 (OAS1, a factor related to the early immune response) expression, on the bottom row (11D to 11F) there are the values of TNC expression. 11A and 11D show the expression when a composition called SYV2 was used that comprised LNPs comprising siRNA and Lipid F. 11B and 11E show a different composition called SYV3 that comprised LNPs comprising siRNA and Lipid F. 11C and 11F show a composition called SYV4 that comprised LNPs comprising siRNA and Lipid O. That means, e.g., 11A shows the OAS1 expression in a formulation comprising the composition called SYV2. The compositions called SYV7, SYV8 and SYV9 comprise LNPs without an active ingredient for comparison and represent control compositions.

**Figure 12 (Fig. 12)** shows the relative cell viability of the HepG2 cell line after 24 hours-incubation with 10, 20, 40, 60, 100, 200, 400, 600, 800 and 1000 μM of ionizable lipids according to the invention.

**Figure 13 (Fig. 13)** shows the relative cell viability of the HepG2 cell line after 48 hours-incubation with 10, 20, 40, 60, 100, 200, 400, 600, 800 and 1000 μM of ionizable lipids according to the invention.

**Figure 14 (Fig. 14)** shows IC50 values of ionizable lipids according to the invention plus, for better illustration, IC50 values of ionizable lipid *SM-102* in comparison after 24 hours and 48 hours, respectively. The higher the value, the better is the stability of the formulation. The better the stability of the formulation, the better can encapsulated molecules be protected.

**Definitions**

The term "salt" as used herein refers to a chemical compound that consisting of cations and anions, which results in a compound with no net electric charge, i.e. that it is electrically neutral. The constituent ions are held together by electrostatic forces termed ionic bonds. The component ions in a salt can be either inorganic or organic.

An "isomer" refers to a chemical compound with the same molecular formula and molecular mass as a compound to be compared, but which differs in the linkage or spatial arrangement of the atoms. Preferably, the term "isomer" as used herein refers to a stereoisomer, such as a configuration isomer or a conformational isomer. A configuration isomer may be an enantiomer or a diastereomer, such as such as cis-trans-isomer, epimer, meso-form, endo-exo-isomer. A configuration isomer may be a rotamer or an atropisomer.

The term "alkyl" or "alkyl group" as used herein refers to a part of a molecule consisting of carbon and hydrogen atoms bonded together. The shortest possible alkyl is a methyl or methyl group. Preferably the term "alkyl" as used herein refers to a part of a molecule consisting of saturated hydrocarbons. A part of a molecule with more than one hydrocarbon may be defined herein as "alkyl chain". An alkyl chain can act as an electron donor due to its +I effect, which may have an effect on the lipophilia of a molecule. Alkyls are often part of a lipid or a lipophilic molecule structure.

The term "alkenyl" or "alkenyl group" or "alkenyl chain" as used herein refers to a part of a molecule consisting of carbon and hydrogen atoms bonded together with one or more double bonds. An alkenyl is an unsaturated part of a molecule, wherein double bonds can be located at any position. There are aliphatic and cyclic alkenyl chains. The shortest possible alkenyl is ethylene or ethylene group. An alkenyl according to the invention includes its isomers, especially its cis-trans isomers. An alkenyl chain may have an effect on the lipophilia of a molecule and is often part of a lipid or a lipophilic molecule structure.

The term "alkinyl" or "alkinyl group" or "alkinyl chain" or "alkyne group" or "alkyne chain" as used herein refers to a part of a molecule consisting of carbon and hydrogen atoms bonded together with one or more triple bonds. An alkinyl is an unsaturated part of a molecule, wherein triple bonds can be located at any position. There are aliphatic and cyclic alkinyl chains. The shortest possible alkinyl is ethyne or ethyne group. An alkinyl according to the invention includes its isomers, especially its cis-trans isomers.

The term "branched" as used herein refers to a part of a molecule consisting of carbon and hydrogen atoms and has at least one side chain. A branched hydrocarbon chain has at least one carbon atom that is bonded to three or more other carbon atoms. A branched hydrocarbon can be chiral if it has a stereocenter, i.e. a carbon atom connected to four different groups. The branching also influences the van der Waals forces that act between the molecules. The longer and straighter the chain, the stronger are the forces of attraction. The shorter and more branched the chain, the weaker are the forces of attraction.

The term "unbranched" as used herein refers to a part of a molecule consisting of carbon and hydrogen atoms without a side chain.

The term "saturated" as used herein refers to a part of a molecule consisting exclusively of single bonds. Saturated hydrocarbons consist exclusively of C-C single bonds. All other possible bonds are already occupied by hydrogen atoms. With more than three carbon atoms, the part of the molecule can be unbranched or branched. Saturated parts of molecules are typical for alkyls.

The term "unsaturated" as used herein refers to a part of a molecule containing at least one double or triple bond that can be located at any position. Unsaturated parts of molecules are typical for alkenyls and alkinyls. Unsaturated hydrocarbons are generally more reactive than saturated hydrocarbons with the same number of carbon atoms.

The term "symmetric" as used herein refers to a hydrocarbon chain which consists of the same number of carbon atoms in comparison to another hydrocarbon chain. As a nonlimiting example, R1 and R2 as used herein can be symmetric alkyls, if they have the same number of carbon atoms and thus can be hydrocarbon chains of equal length.

The term "asymmetric" as used herein refers to a hydrocarbon chain which consists of a different number of carbon atoms in comparison to another hydrocarbon chain. As a nonlimiting example, R1 and R2 as used herein can be asymmetric alkyls, if they have a different number of carbon atoms and thus can be hydrocarbon chains of different length.

The term "ionizable" as used herein refers to the capability of a chemical compound, atom or molecule to get a positive charge or a negative charge. Herein a positive charge is preferred. Removing one or more electrons of an atom or a molecule leads to a cation, which is positively charged. Attachment of an electron to a neutral atom or molecule leads to an anion, which is negatively charged. Ejection of the nucleus of an atom from the electron shell can also lead to a charged ion.

The term "ionizable lipid" as used herein refers to a compound with lipophilic chemical structure, which is ionizable.

The term "ionizable lipid nanoparticle" or "ionizable LNP" or "iLNP" as used herein refers to a nanoparticle comprising an ionizable lipid. An ionizable LNP can comprise further lipid components. It may include at least another ionizable lipid, PEGylated lipids, phopspholipids, structural lipids and other lipids, such as SORT lipids.

The term "phospholipid" as used herein refers to a lipid comprising at least one phosphate moiety and at least one carbon chain, such as an unsaturated fatty acid chain. Phospholipids may have a quaternized amine headgroup and a

lipophilic tail group. They may help promoting fusion with the target cell and possibly with endosomes. Phospholipids can increase the phase-transition temperature and form lipid bilayer structures of iLNPs.

The term "hydrophilic" as used herein refers to a molecule or a part of a molecule that interacts preferably with water or other polar substances than with non-polar substances and tends to solvate in water.

The term "lipophilic" as used herein refers to a molecule or a part of a molecule that preferably dissolves in fats and oils than in water or can itself dissolve fats and oils better than water.

The term "PEGylated" as used herein refers to a chemical residue of a molecule comprising a polyethylene glycol group. The term "PEGylated lipid" or "polyethylene glycol lipid" as used herein refers to a lipid comprising a polyethylene glycol group. PEGylated lipids generally improve the hydrophilicity of iLNPs and thus also colloidal stability, stabilize the structures of iLNPs, prevents nanoparticles aggregation, avoid rapid clearance of the molecule to be delivered by the target organism, and increase circulation time in vivo and reduce clearance.

The term "structure lipid" or "structural lipid" as used herein refers to lipids with a membrane fusion function. They are made to promote the uptake of molecules, such as mRNA, into cytoplasm and to enhance nanoparticle stability. Cholesterol and sitosterol are examples for lipids with the function of a structure lipid.

The term "SORT lipid" or "Selective organ targeting lipid" as used herein refers to mostly phospholipids with charge, including permanent cationic lipids, negatively charged lipids, and other ionizable lipids. SORT lipids may affect the in vivo distribution.

When a part of a molecule is "connected to" another part of a molecule or a chemical residue as used herein, a chemical bonding is described. The sort of chemical bonding includes covalent bonds, ionic bonds, metallic bonds, hydrogen bonds and Van der Waals interactions as well as connecting moieties. E.g., when a molecule or a part of a molecule is connected to another molecule or part of a molecule via an alkyl, the alkyl is the connecting moiety that connects the molecules or molecule parts.

The term "pharmaceutically acceptable" is used herein to refer to those compounds, materials, compositions, and/or dosage forms which are, within the scope of sound medical judgment, suitable for use in contact with the tissues of human beings and animals without excessive toxicity, allergic response, irritation, or other problem or complication, commensurate with a reasonable benefit-risk ratio.

The term "tail group" as used herein refers to a chemical part of the molecule which can be considered the lipophilic part of the molecule. Usually the tail group is a hydrocarbon chain, preferably an alkyl or alkenyl.

The term "head group" as used herein refers to a chemical part of the molecule which can be considered ionizable. It may comprise a hetero atom, preferably an ionizable nitrogen atom.

The term "core" as used herein refers to a chemical moiety which connects the head group to the tail group of a molecule.

The term "patient" as used herein refers to a subject who may seek or be in need of treatment, requires treatment, is receiving treatment, will receive treatment, or a subject who is under care by a trained professional for a particular disease or condition.

The term "PC" as used herein refers to phosphatidylcholine. The term "GMO" as used herein refers to a genetically modified organism. The term "NON-GMO" as used herein refers to an organism that is not genetically modified. The term "DSPC" as used herein refers to 1,2-distearoyl-sn-glycero-3-phosphocholine. The term "DPPC" as used herein refers to 1,2-dipalmitoyl-sn-glycero-3-phosphocholine. The term "HSPC" as used herein refers to hydrogenated soybean phosphatidylcholine. The term "HEPC" as used herein refers to hydrogenated egg yolk phosphatidylcholine. The term "DLPC" as used herein refers to 1,2-dilinoleoyl-sn-glycero-3-phosphocholine. The term "DMPC" as used herein refers to 1,2-dimyristoyl-sn-glycero-phosphocholine. The term "DOPC" as used herein refers to 1 ,2-dioleoyl-sn-glycero-3-phosphocholine. The term "DUPC" as used herein refers to 1,2-diundecanoyl-sn-glycero-phosphocholine. The term "POPC" as used herein refers to 1-palmitoy 1-2-oleoy 1-sn-glycero-3-phosphocholine. The term "SOPC" as used herein refers to 1 -stearoyl-2-oleoylphosphatidylcholine. The term "ePC" as used herein refers to egg yolk phosphatidylcholine. The term "sPC" as used herein refers to soybean phosphatidylcholine. The term "hPC" as used herein refers to sunflower phosphatidylcholine. The term "PE" as used herein refers to phosphatidylethanolamine. The term "DOPE" as used herein refers to 1,2-dioleoyl-sn-glycero-3-phosphoethanolamine. The term "POPE" as used herein refers to palmitoyloleoylphosphatidylethanolamine. The term "DPPE" as used herein refers to dipalmitoylphosphatidylethanolamine. The term "DMPE" as used herein refers to dimyristoylphosphoethanolamine. The term "SOPE" as used herein refers to 1-stearoyl-2-oleoyl-phosphatidyethanolamine. The term "LPE" as used herein refers to lysophosphatidylethanolamine. The term "PG" as used herein refers to phosphatidylglycerol. The term "DSPG" as used herein refers to 1,2-Distearoyl-sn-glycero-3-phosphoglycerol. The term "DOPG" as used herein refers to 1,2-dioleoyl-sn-glycero-3-phospho-1-glycerol. The term "DPPG" as used herein refers to dipalmitoylphosphatidylglycerol. The term "PA" as used herein refers to phosphatidic acid. The term "DPPA" as used herein refers to 1,2-Dipalmitoyl-sn-glycero-3-phosphatidic acid. The term "PI" as used herein refers to phosphatidylinositol. The term "PS" as used herein refers to phosphatidylserine. The term "DSPS" as used herein refers to 1,2-Distearoyl-sn-glycero-3-phospho-L-serine. The term "LPL" as used herein refers to monoacyl phospholipids such as lysophospholipid.

The term "DMG-PEG" as used herein refers to 1,2-dimyristoyl-sn-glycerol methoxypolyethyleneglycol. The term "DSPE-PEG" as used herein refers to 1,2-distearoyl-sn-glycero-3-phosphoethanolamine-N-[amino(polyethylene glycol)]. The term "DSG-PEG" as used herein refers to disteryl glycerol polyethyleneglycol. The term "PEG-CER" as used herein refers to PEGylated ceramide. The term "PEG-DEG" as used herein refers to PEGylated diacylglycerol. The term "PEG-DMPE" as used herein refers to PEGylated dimyristoylphosphoethanolamine. The term "PEG-DPPC" as used herein refers to PEGylated DPPC. The term "PEG-c-DOMG" as used herein refers to [(Methoxy-poly(ethyleneglycol) 2000)carbamoyl]-1,2-dimyristyloxlpropyl-3-amine. The term "DPPE-PEG" as used herein refers to PEGylated DPPE. The term "c-DMA-PEG" as used herein refers to PEGylated 1,2-dimyristoyl-sn-glycerol. The term "pSar" as used herein refers to polysarcosine. The term "DMG-pSar25" as used herein refers to 1 ,2-dimyristoyl-sn-glycero-3-succinyl-N-polysarcosine-25. The term "DOPE-pSar" as used herein refers to 1,2-dioleoyl-sn-glycero-3-phosphoethanolamine-N-polysarcosine-25.

The term "DODAP" as used herein refers to 1,2-dioleoyl-3-dimethylammonium-propane. The term "DOTAP" as used herein refers to 1,2-dioleoyl-3-trimethylammonium-propane. The term "DPDAB" as used herein refers to 1 ,2-dipalmitoyl-3-dimethylammonium-propane. The term "DODMA" as used herein refers to 1,2-dioleyloxy-3-dimethylaminopropane. The term "14PA" as used herein refers to 1,2-dimyristoyl-sn-glycero-3-phosphate. The term "18BMP" as used herein refers to bis(monooleoylglycero)phosphate. The term "18PA" as used herein refers to 1,2-dioleoyl-sn-glycero-3-phosphate. The term "DC-cholesterol" as used herein refers to dimethylaminoethane-carbamoyl-cholesterol. The term "5A2-SC8" as used herein refers to a compound defined by CAS number 1857341-90-2.

The term "MC3" as used herein refers to the lipid *D-Lin-MC3-DMA.* The term "C12" as used herein refers to the lipid *C12-200.*

**Detailed description of the invention**

The present invention provides novel compounds suitable as ionizable lipids, even representing a novel class of ionizable lipids. It further provides ionizable lipid nanoparticles and compositions related thereto and uses thereof. The key contribution of the invention is based on a benzene core with oxygen residues, especially a hydroxybenzoic acid structure, which is particularly effective for the formation of LNPs used in molecule delivery. The core moiety may have chemical residues in ortho-, meta- and/or para-position which offers different ionisation potentials. The lipids according to the invention are moreover characterised by the presence of specific alkyl chains connected to a carboxyl group to increase the hydrophobicity of the lipid and the stability of the resulting. A further main contribution is a tertiary amine group which is evident for the capability of being ionized.

The present invention provides completely new chemical structural classes of compounds suitable as ionizable lipids and thus expands the possibilities in the field of LNP technology tremendously. By efficiently protecting and delivering molecules to specific locations of various target organism, bioavailability can be enhanced, while the biodegradable compounds lead to lower toxicity for the respective target organism. A wide range of uses of such compounds, ionizable lipids, LNPs and compositions according to the invention is the result.

To this end, the present invention provides, in a first aspect, a compound being represented by formula (A) or formula (B) or a salt or isomer thereof, preferably being suitable as lipid, particularly preferably being used as ionizable lipid

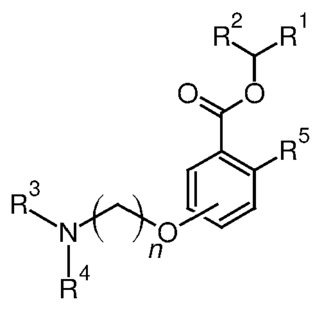

formula (A)

wherein $R_1$ is an alkyl, an alkenyl or an alkinyl,

wherein $R_2$ is selected from the group consisting of H, COOH, benzyl, phenol, an alkyl, and an alkenyl,

wherein $R_3$ and $R_4$ are independently selected from the group consisting of H, $NH_2$, benzyl, phenol, benzoic acid, an alkyl, and an alkenyl,

wherein $R_5$ is selected from the group consisting of H, $NH_2$, an alkyl, an alkenyl or an alkinyl,

wherein n is 1, 2, 3, 4, 5 or 6, preferably 1, 2, 3 or 4, more preferably 2 or 3, particularly preferably 2,

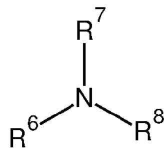

formula (B),

wherein $R_6$ is represented by formula (B-1)

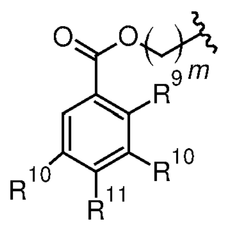

formula (B-1),

or a salt or isomer thereof,

wherein $R_7$ is represented by formula (B-1) or a salt or isomer thereof, or is selected from the group consisting of H, an alkyl with a total number of carbon atoms in the range from 1 to 7, a methanol group, an ethanol group, a propanol group, a butanol group or a pentanol group, preferably a methyl group,

wherein $R_8$ is represented by formula (B-1) or a salt or isomer thereof, or is selected from the group consisting of H, an alkyl with a total number of carbon atoms in the range from 1 to 7, a methanol group, an ethanol group, a propanol group, a butanol group or a pentanol group, preferably a methyl group or a propanol group,

wherein $R_9$ is selected from the group consisting of H, O-$R_{B1}$ or an alkyl,

wherein $R_{B1}$ is selected from the group consisting of H, OH or an alkyl,

wherein $R_{10}$ is selected from the group consisting of H, O-$R_{B2}$ or an alkyl,

wherein $R_{B2}$ is selected from the group consisting of H, OH or an alkyl

wherein $R_{11}$ is selected from the group consisting of H, O-$R_{B3}$ or an alkyl,

wherein $R_{B3}$ is selected from the group consisting of H, OH or an alkyl, and/or

wherein m is 1, 2, 3, 4, 5 or 6, preferably 2, 3, 4 or 5, particularly preferably 2 or 5,

preferably wherein at least one of $R_9$, $R_{10}$ or $R_{11}$ is selected from the group consisting of $OR_{B1}$, O-$R_{B2}$ or O-$R_{B3}$.

In one embodiment the alkyl or alkenyl or alkinyl representing $R_1$ may have a total number of carbon atoms of 1, 2, 3, 4, 5, 6, 7, 8, 9, 10, 11, 12, 13, 14, 15, 16, 17, 18, 19, 20, 21, 22, 23, 24, 25, or above.

In a preferred embodiment, $R_1$ may be an alkyl with a total number of carbon atoms in the range from 5 to 17, more preferably an alkyl with a total number of carbon atoms in the range from 8 to 15, particularly preferably an alkyl with a total number of carbon atoms in the range from 10 to 13,

In one embodiment, $R_2$ may be H. In another embodiment, $R_2$ may be an alkyl with a total number of carbon atoms

in the range from 5 to 17, preferably an alkyl with a total number of carbon atoms in the range from 8 to 15, particularly preferably an alkyl with a total number of carbon atoms in the range from 10 to 13.

In yet another embodiment, $R_2$ may be an alkenyl with a total number of carbon atoms in the range from 1 to 20.

In one embodiment, $R_3$ may be an alkyl with a total number of carbon atoms in the range from 1 to 7, preferably an alkyl with a total number of 1 or 2 carbon atoms, particularly preferably a methyl group.

In another embodiment, $R_3$ may be an alkenyl with a total number of carbon atoms in the range from 1 to 10.

In one embodiment, $R_4$ may be an alkyl with a total number of carbon atoms in the range from 1 to 7, preferably an alkyl with a total number of 1 or 2 carbon atoms, particularly preferably a methyl group.

In another embodiment $R_4$ may be an alkenyl with a total number of carbon atoms in the range from 1 to 10.

In one embodiment, the alkyl representing $R_3$ can be a cyclic structure to connect $R_3$ and Ra.

In one embodiment, $R_3$ and $R_4$ can be connected to the tertiary amine to represent a pyrrolidine. In another embodiment, $R_3$ and $R_4$ can be connected to the tertiary amine to represent a piperidine.

In another embodiment, the alkenyl representing $R_3$ can be a cyclic structure to connect $R_3$ and $R_4$.

In one embodiment, $R_5$ may be H. In another embodiment, $R_5$ may be an alkyl with a total number of carbon atoms in the range from 1 to 25, preferably an alkyl with a total number of carbon atoms in the range from 10 to 19, more preferably an alkyl with a total number of carbon atoms in the range from 12 to 18, particularly preferably an alkyl with a total number of 17 carbon atoms.

In yet another embodiment, $R_5$ may be an alkenyl with a total number of carbon atoms in the range from 1 to 25. In a further embodiment, $R_5$ may be an alkinyl with a total number of carbon atoms in the range from 1 to 25.

According to the present disclosure, $R_1$, $R_2$, $R_3$, $R_4$ and $R_5$ may be individually selected according to the embodiments detailed above, unless specific combinations are individually provided.

In one embodiment, $R_7$ may be represented by formula (B-1). In another embodiment, $R_7$ may be an alkyl with a total number of carbon atoms in the range from 1 to 7, preferably a methyl group.

In yet another embodiment, $R_7$ may be an alkenyl with a total number of carbon atoms in the range from 1 to 7.

In one embodiment, the alkyl representing $R_7$ can be a cyclic structure to connect $R_7$ and $R_8$. Thus, a cyclic tertiary amine can be represented herein.

In another embodiment, the alkenyl representing $R_7$ can be a cyclic structure to connect $R_7$ and $R_8$. Thus, an aromatic tertiary amine can be represented herein.

In one embodiment, $R_8$ may be represented by formula (B-1). In another embodiment $R_8$ may be an alkyl with a total number of carbon atoms in the range from 1 to 7, preferably a methyl group or a propanol group.

In yet another embodiment, $R_8$ may be an alkenyl with a total number of carbon atoms in the range from 1 to 7.

In one embodiment, the alkyl representing $R_8$ can be a cyclic structure to connect $R_7$ and $R_8$. Thus, a cyclic tertiary amine can be represented herein.

In another embodiment, the alkenyl representing $R_8$ can be a cyclic structure to connect $R_7$ and $R_8$. Thus, an aromatic tertiary amine can be represented herein.

According to the present disclosure, $R_6$, $R_7$ and $R_8$ may be individually selected according to the embodiments detailed above, unless specific combinations are individually provided.

In one embodiment, Rg may be H. In another embodiment, Rg may be O-$R_{B1}$.

In one embodiment, $R_{B1}$ may be H. In another embodiment, $R_{B1}$ may be an alkyl with a total number of carbon atoms of 1, 2, 3, 4, 5, 6, 7, 8, 9, 10, 11, 12, 13, 14, 15, 16, 17, 18, 19, 20, 21, 22, 23, 24, 25, or above. In a preferred embodiment, $R_{B1}$ may be an alkyl with a total number of carbon atoms in the range from 10 to 20, more preferably an alkyl with a total number of carbon atoms in the range from 15 to 18, particularly preferably an alkyl with a total number of 16 carbon atoms.

In one embodiment, $R_{10}$ may be H. In another embodiment, $R_{10}$ may be O-$R_{B2}$.

In one embodiment, $R_{B2}$ may be H. In another embodiment, $R_{B2}$ may be an alkyl with a total number of carbon atoms of 1, 2, 3, 4, 5, 6, 7, 8, 9, 10, 11, 12, 13, 14, 15, 16, 17, 18, 19, 20, 21, 22, 23, 24, 25, or above. In a preferred embodiment, $R_{B2}$ may be an alkyl with a total number of carbon atoms in the range from 10 to 20, more preferably an alkyl with a total number of carbon atoms in the range from 12 to 16, particularly preferably an alkyl with a total number of 12 or 16 carbon atoms.

In one embodiment, $R_{11}$ may be H. In another embodiment, $R_{11}$ may be O-$R_{B2}$.In yet another embodiment, $R_{11}$ may be an alkyl with a total number of carbon atoms of 1, 2, 3, 4, 5, 6, 7, 8, 9, 10, 11, 12, 13, 14, 15, 16, 17, 18, 19, 20, 21, 22, 23, 24, 25, or above. In a preferred embodiment, $R_{11}$ may be an alkyl with a total number of carbon atoms in the range from 10 to 20, preferably an alkyl with a total number of carbon atoms in the range from 15 to 18, particularly preferably an alkyl with a total number of 15 or 18 carbon atoms.

In one embodiment, $R_{B3}$ may be H. In another embodiment, $R_{B3}$ may be an alkyl with a total number of carbon atoms of 1, 2,b 3, 4, 5, 6, 7, 8, 9, 10, 11, 12, 13, 14, 15, 16, 17, 18, 19, 20, 21, 22, 23, 24, 25, or above. In a preferred embodiment, $R_{B3}$ may be an alkyl with a total number of carbon atoms in the range from 10 to 20, more preferably an alkyl with a total number of carbon atoms in the range from 15 to 18, particularly preferably an alkyl with a total number of 15 or 16

carbon atoms.

According to the present disclosure, $R_9$, $R_{10}$ and $R_{11}$ may be individually selected according to the embodiments detailed above, unless specific combinations are individually provided.

It was advantageously found that the lipid chains represented by $R_1$, $R_2$, $R_5$, $R_4$, $R_5$, $R_9$, $R_{10}$, $R_{11}$, $R_{B1}$, O-$R_{B2}$ and O-$R_{B3}$, especially $R_1$, $R_2$, $R_5$, $R_9$, $R_{10}$, $R_{11}$, $R_{B1}$, O-$R_{B2}$ and O-$R_{B3}$, are critical to the stability of the resulting compounds, ionizable lipids and/or LNPs. They may have a certain chain length to increase the hydrophobicity of the lipid. Moreover, these tail groups may be characterised by the presence of specific alkyl chains, alkenyl chains or alkinyl chains, preferably alkyl chains, that are connected to a carboxyl group, preferably to the oxygen atom of an ester group, which represents a feature of a completely novel class of ionizable lipids or compounds being suitable therefore.

In a preferred embodiment, there is provided a compound according to the various aspects, wherein the at least one tertiary amine may be a tertiary aliphatic amine. In another embodiment, there is provided a compound according to the various aspects, wherein the at least one tertiary amine may be a tertiary cyclic amine. In one embodiment the tertiary cyclic amine may be represented by a pyrrolidine. In another embodiment the tertiary cyclic amine may be represented by a piperidine. In yet another embodiment, there is provided a compound according to the various aspects, wherein the at least one tertiary amine may be a tertiary aromatic amine. A tertiary amine group is evident for the capability of being ionized. The different embodiments may have an effect on the basic quantity of the tertiary amine and thus be ionized in different circumstances.

In another embodiment, there is provided a compound according to the various aspects, wherein further at least one of $R_1$, $R_2$, $R_5$, $R_9$, $R_{10}$, $R_{11}$, $R_{B1}$, $R_{B2}$ and $R_{B3}$ is/are an alkyl, alkenyl or an alkinyl with a total number of carbon atoms of 1, 2, 3, 4, 5, 6, 7, 8, 9, 10, 11, 12, 13, 14, 15, 16, 17, 18, 19, 20,21,22, 23, 24, 25, or above, preferably with a total number of carbon atoms in the range from 6 to 24, more preferably wherein at least one of $R_1$, $R_2$, $R_5$, $R_9$, $R_{10}$, $R_{11}$, $R_{B1}$, $R_{B2}$ and $R_{B3}$ has a total number of carbon atoms of 10, 12, 13, 15, 16, 17 or 18.

In one embodiment, the alkyl representing at least one of $R_1$, $R_2$, $R_5$, $R_9$, $R_{10}$, $R_{11}$, $R_{B1}$, $R_{B2}$ and $R_{B3}$ is branched. In another embodiment, the alkyl representing at least one of $R_1$, $R_2$, $R_5$, $R_9$, $R_{10}$, $R_{11}$, $R_{B1}$, $R_{B2}$ and $R_{B3}$ is unbranched.

In one embodiment, the alkenyl representing at least one of $R_1$, $R_2$, $R_5$, $R_9$, $R_{10}$, $R_{11}$, $R_{B1}$, $R_{B2}$ and $R_{B3}$ is branched. In another embodiment, the alkenyl representing at least one of $R_1$, $R_2$, $R_5$, $R_9$, $R_{10}$, $R_{11}$, $R_{B1}$, $R_{B2}$ and $R_{B3}$ is unbranched.

In one embodiment, the alkinyl representing at least one of $R_1$, $R_2$, $R_5$, $R_9$, $R_{10}$, $R_{11}$, $R_{B1}$, $R_{B2}$ and $R_{B3}$ is branched. In another embodiment, the alkinyl representing at least one of $R_1$, $R_2$, $R_5$, $R_9$, $R_{10}$, $R_{11}$, $R_{B1}$, $R_{B2}$ and $R_{B3}$ is unbranched.

In one embodiment, the alkyl representing at least one of $R_1$, $R_2$, $R_5$, $R_9$, $R_{10}$, $R_{11}$, $R_{B1}$, $R_{B2}$ and $R_{B3}$ is saturated. In another embodiment, the alkyl representing at least one of $R_1$, $R_2$, $R_5$, $R_9$, $R_{10}$, $R_{11}$, $R_{B1}$, $R_{B2}$ and $R_{B3}$ is unsaturated.

In one embodiment, the alkenyl representing at least one of $R_1$, $R_2$, $R_5$, $R_9$, $R_{10}$, $R_{11}$, $R_{B1}$, $R_{B2}$ and $R_{B3}$ is saturated. In another embodiment, the alkenyl representing at least one of $R_1$, $R_2$, $R_5$, $R_9$, $R_{10}$, $R_{11}$, $R_{B1}$, $R_{B2}$ and $R_{B3}$ is unsaturated.

In one embodiment, the alkinyl representing at least one of $R_1$, $R_2$, $R_5$, $R_9$, $R_{10}$, $R_{11}$, $R_{B1}$, $R_{B2}$ and $R_{B3}$ is saturated. In another embodiment, the alkinyl representing at least one of $R_1$, $R_2$, $R_5$, $R_9$, $R_{10}$, $R_{11}$, $R_{B1}$, $R_{B2}$ and $R_{B3}$ is unsaturated.

In one embodiment, the alkyl representing at least one of $R_1$, $R_2$, $R_5$, $R_9$, $R_{10}$, $R_{11}$, $R_{B1}$, $R_{B2}$ and $R_{B3}$ is symmetric. In another embodiment, the alkyl representing at least one of $R_1$, $R_2$, $R_5$, $R_9$, $R_{10}$, $R_{11}$, $R_{B1}$, $R_{B2}$ and $R_{B3}$ is asymmetric.

In one embodiment, the alkenyl representing at least one of $R_1$, $R_2$, $R_5$, $R_9$, $R_{10}$, $R_{11}$, $R_{B1}$, $R_{B2}$ and $R_{B3}$ is symmetric. In another embodiment, the alkenyl representing at least one of $R_1$, $R_2$, $R_5$, $R_9$, $R_{10}$, $R_{11}$, $R_{B1}$, $R_{B2}$ and $R_{B3}$ is asymmetric.

In one embodiment, the alkinyl representing at least one of $R_1$, $R_2$, $R_5$, $R_9$, $R_{10}$, $R_{11}$, $R_{B1}$, $R_{B2}$ and $R_{B3}$ is symmetric. In another embodiment, the alkinyl representing at least one of $R_1$, $R_2$, $R_5$, $R_9$, $R_{10}$, $R_{11}$, $R_{B1}$, $R_{B2}$ and $R_{B3}$ is asymmetric.

In yet another embodiment, there is provided a compound according to the various aspects, wherein further the alkyl representing at least one of $R_1$, $R_2$, $R_5$, $R_9$, $R_{10}$, $R_{11}$, $R_{B1}$, $R_{B2}$ and $R_{B3}$ can comprise one or more hetero atoms as chemical residue. In a preferred embodiment the hetero atom(s) is/are oxygen atom(s). In a further embodiment, the hetero atom(s) is/are nitrogen atom(s). In yet a further embodiment, the hetero atom(s) is/are halogen atom(s), selected from a group consisting of F, Cl, Br, I and At.

In yet another embodiment, there is provided a compound according to the various aspects, wherein further the alkenyl representing at least one of $R_1$, $R_2$, $R_5$, $R_9$, $R_{10}$, $R_{11}$, $R_{B1}$, $R_{B2}$ and $R_{B3}$ can comprise one or more hetero atoms as chemical residue. In a preferred embodiment the hetero atom(s) is/are oxygen atom(s). In a further embodiment, the hetero atom(s) is/are nitrogen atom(s). In yet a further embodiment, the hetero atom(s) is/are halogen atom(s), selected from a group consisting of F, Cl, Br, I and At.

In yet another embodiment, there is provided a compound according to the various aspects, wherein further the alkinyl representing at least one of $R_1$, $R_2$, $R_5$, $R_9$, $R_{10}$, $R_{11}$, $R_{B1}$, $R_{B2}$ and $R_{B3}$ can comprise one or more hetero atoms as chemical residue. In a preferred embodiment the hetero atom(s) is/are oxygen atom(s). In a further embodiment, the hetero atom(s) is/are nitrogen atom(s). In yet a further embodiment, the hetero atom(s) is/are halogen atom(s), selected

from a group consisting of F, Cl, Br, I and At.

In a further embodiment, there is provided a compound according to the various aspects being represented by formula (1), (2), (3), (4), (5), (6), (7), (8), (9), (10), (11), (12), (13), (14), (15), (16) or (17) or a salt or isomer thereof, preferably being suitable as lipid, particularly preferably being used as ionizable lipid

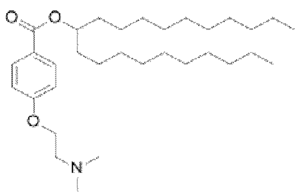

formula (1)

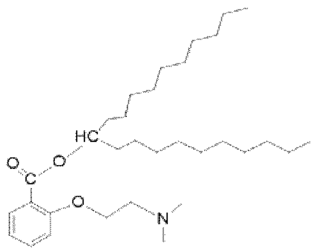

formula (2)

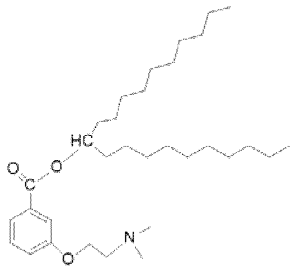

formula (3)

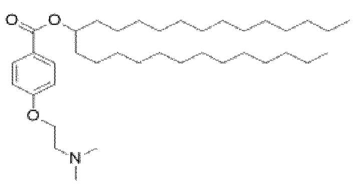

formula (4)

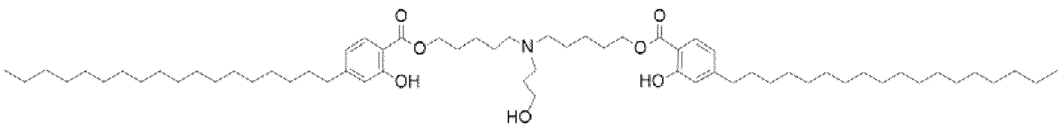

formula (5)

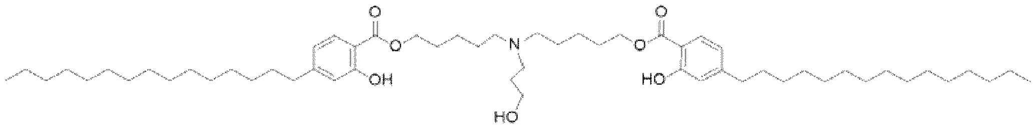

formula (6)

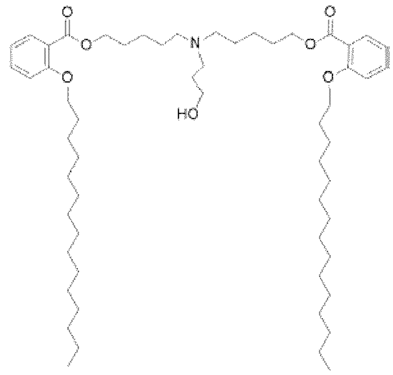

formula (7)

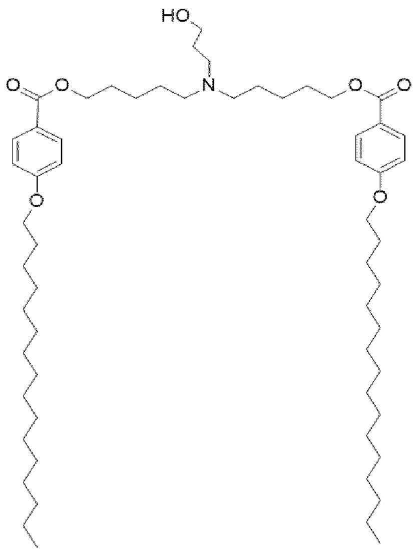

formula (8)

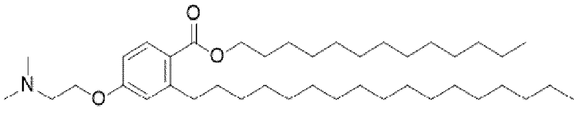

formula (9)

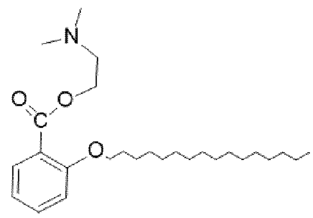

formula (10)

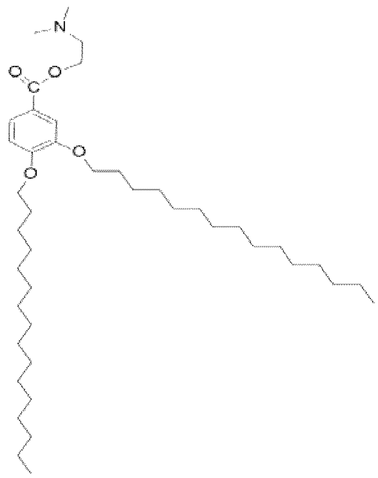

formula (11)

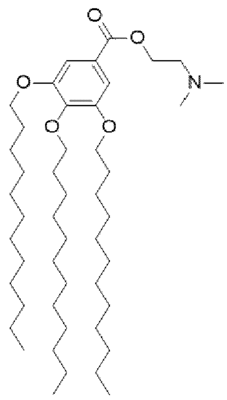

formula (12)

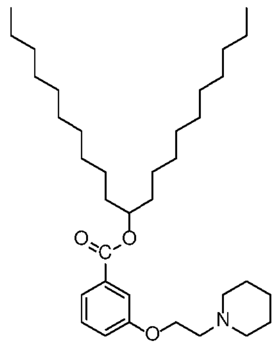

formula (13)

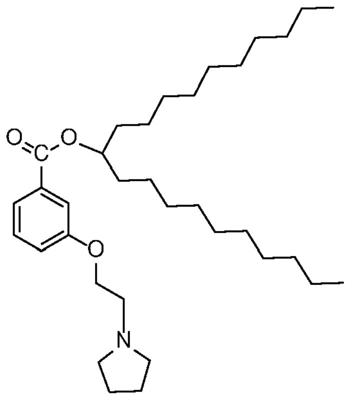

formula (14)

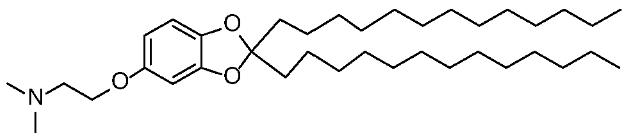

formula (15)

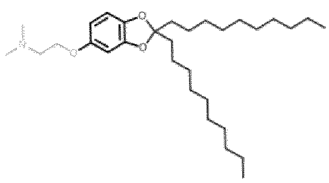

formula (16)

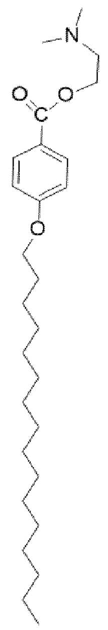

formula (17).

Surprisingly it was found that the compounds according to the invention are able to reduce toxicity for the target organism, as it can be seen in Fig. 12, Fig. 13, Fig 14 and Fig. 4.

The compounds according to the invention, may be suitable as autonomous main ingredient. Preferably, they can be a carrier or an excipient.

Advantageously the compounds being suitable as ionizable lipids can increase the stability of LNP formulations and thus protect the main ingredient and the formulation itself. This is a key contribution to deliver molecules to a specific location of a target organism, as it can be seen in Fig 1, Fig. 2 and Fig. 3.

Further, there was surprisingly found the compounds being suitable as ionizable lipid being highly bioavailable and easily tissue compatible. Therefore, they can be used effectively in several fields of operations, examples can be seen in Fig. 5, Fig. 6, Fig. 7 and Fig. 11. Apart from the use in immune modulation or drug targeting, they can be used in further application areas such as cosmetics, nutrition and the agricultural sector The compounds according to the invention are moreover highly functional and are able to effectively deliver main ingredients to provide a desired effect, as it can be seen in Fig 8, Fig. 9 and Fig. 10.

In a second aspect, there is provided an ionizable lipid according to the various aspects, comprising a hydroxybenzoic acid structure,

preferably wherein the ionizable lipid has a chemical structure of formula (A) or (B), wherein formula (B) includes formula (B-1), as defined above.

In a preferred embodiment, one hydroxybenzoic acid is present.

In another embodiment, two hydroxybenzoic acids are present. In yet another embodiment, three hydroxybenzoic acids are present.

The core of the compound being suitable as an ionizable lipid based on a benzene core with oxygen residues is particularly effective for the formation of LNPs used in molecule delivery.

In certain embodiments the core structure is represented by a hydroxybenzene moiety, like e.g. hydroxylhydroquinone. A preferred embodiment features a hydroxybenzoic acid as core of the compound according to the invention. The core moiety may have chemical residues in ortho-, meta- and/or para-position. The invention includes variations where the hydroxyl-and carboxyl-substituents are in ortho-, meta- or para-positions on the benzene ring of the hydroxybenzoic acid core. These positional isomers offer different ionisation potentials and can be selected based on the desired release profile of the encapsulated drug.

In one embodiment, there is provided an ionizable lipid according to the various aspects, wherein at least one tertiary amine is present and wherein the at least one tertiary amine has at least one chemical residue, wherein the chemical residue is selected from the group consisting of H, an alkyl, a methanol group, an ethanol group, a propanol group, a butanol group or a pentanol group, preferably a methyl group or a propanol group.

In a preferred embodiment the tertiary amine is connected to the phenolic oxygen atom of the hydroxybenzoic acid via an alkyl group, or the tertiary amine is connected to the oxygen atom of the carbonyl group of the hydroxybenzoic acid via an alkyl group.

In a preferred embodiment the carboxyl group of the hydroxybenzoic acid is connected to the tertiary amine via an alkyl group.

In a preferred embodiment the aromatic group of the hydroxybenzoic acid has H, an ether or an alkyl as a chemical residue.

In one embodiment the tail group as chemical residue of the ether and/or the aromatic group of the hydroxybenzoic acid and/or the oxygen atom of the carboxyl group is an alkyl, an alkenyl or an alkinyl with a total number of carbon atoms of 1, 2, 3, 4, 5, 6, 7, 8, 9, 10, 11, 12, 13, 14, 15, 16, 17, 18, 19, 20, 21, 22, 23, 24, 25, or above, preferably with a total number of carbon atoms in the range from 6 to 24, particularly preferably with a total number of carbon atoms of 10, 12, 13, 15, 16, 17 or 18.

In a third aspect, there is provided a method of producing the compound according to the first aspect and the various embodiments related thereto, or the lipid according to the second aspect and the embodiments related thereto, wherein the method comprises the following steps, in sequence:

a) providing a raw material comprising a structure of hydroxbenzoic acid or a derivative or a salt thereof,

b) mixing the provided raw material with a substance for elongating the tail group of the provided raw material,

c) adding a substance to the mixture obtained in b) for hydrating a derivative of a hydroxyl group of the mixture obtained in b),

d) adding a substance comprising a tertiary amine to the mixture obtained in c), and

e) obtaining a product comprising a hydroxybenzoic acid structure connected to a tertiary amine and further comprising a lipophilic tail group.

In a fourth aspect, there is provided an ionizable lipid nanoparticle comprising the compound according to the first aspect and the various embodiments related thereto or the lipid according to the second aspect and the embodiments related thereto.

In one embodiment, there is provided an ionizable lipid nanoparticle according to the various aspects, further comprising

structure lipids, wherein the structure lipid is phospholipid and/or sphingolipid, preferably wherein the phospholipid is selected from the group consisting of PC, PE, PA, PG, PI, PS and LPL, preferably wherein the sphingolipid is sphingomyelin, more preferably wherein the phospholipid is selected from the group of DSPC, DOPE, HSPC, DPPC, DSPG, DSPS and DPPA,

sterols, preferably wherein the sterol is cholesterol and/or sitosterol,

lipids modified with hydrophilic polymers, preferably wherein the lipid modified with hydrophilic polymers is PEGylated lipid, more preferably wherein the PEGylated lipid is DMG-PEG and/or DSPE-PEG and/or DSG-PEG,

and optionally

SORT lipids, preferably wherein the SORT lipid comprises DODAP, DOTAP, DPDAB, DODMA, 14PA, 18BMP, 18PA, DC-cholesterol and/or 5A2-SC8,

and optionally

antioxidants and/or oils and/or fatty acids and/or fatty acid salts and/or surfactants.

In one embodiment the phospholipid may be connected to palmitoyloleoyl. In a certain embodiment PC may be of plant origin, such as GMO and NON-GMO. In another embodiment PC may be of animal origin. In yet another embodiment PC may be of synthetic origin. In one embodiment PC may be lecithin. In a certain embodiment lecithin is selected from the group consisting of soybean lecithin, sunflower lecithin and rapeseed lecithin.

In one embodiment PC is selected from the group consisting of DSPC, DPPC, HSPC, HEPC, DLPC, DEPC, DMPC, DOPC, DUPC, POPC, SOPC, ePC, sPC and hPC. In a preferred embodiment PC is DSPC and/or HSPC and/or DPPC. In one embodiment PE is selected from the group consisting of DOPE, POPE, DPPE, DMPE, SOPE and LPE. In a preferred embodiment PE is DOPE. In one embodiment PA is DPPA. In one embodiment PG is DSPG and/or DOPG and/or

DPPG. In a preferred embodiment PG is DSPG. In one embodiment PS is DSPS.

In one embodiment the origin of the sterol is selected from the group consisting of plant, animal, fungi and synthetic. In a certain embodiment the sterol of animal origin is selected from the group consisting of cholesterol, lanosterol, koprosterol, cholestanol, lithocholic acid and cholic acid. In another embodiment the sterol of plant origin is selected from the group consisting of cholesterol, sitosterol, stigmasterol, campesterol and brassikasterol. In a further embodiment the sterol of fungi origin is ergosterol.

In one embodiment the lipid modified with hydrophilic polymers is selected from the group consisting of PEGylated diglyceride, PEGylated phospholipid, PEGylated sterol, PEG with a linker, lipid with lactic acid modification, lipid with polylactic acid modification, lipid with polylactic-co-glycolic acid modification, polysaccharide modified lipid and pSar lipid. In one embodiment the lipid modified with hydrophilic polymers comprises PEG 600. In another embodiment the lipid modified with hydrophilic polymers comprises PEG 2000. In yet another embodiment the lipid modified with hydrophilic polymers comprises PEG 5000. In one embodiment the PEGylated lipid is selected from the group consisting of DMG-PEG, DSPE-PEG, DSG-PEG, PEG-CER, PEG-DEG, PEG-DMPE, PEG-DPPC, PEG-c-DOMG, DPPE-PEG and c-DMA-PEG. In a preferred embodiment the PEGylated lipid is DMG-PEG and/or DSPE-PEG and/or DSG-PEG. In one embodiment the lipid modified with hydrophilic polymers comprises DMG-pSar25. In one embodiment the lipid modified with hydrophilic polymers comprises DOPE-pSar.

In one embodiment the antioxidant is selected from the group consisting of Vitamin E and its derivatives such as alpha-tocopherol, beta-tocopherol, gamma-tocopherol, tocotrienol and mixes thereof, carotenoids such as beta-carotene, lycopene, lutein and astaxanthin, resveratrol, lipoic acid and ubiquinone.

In one embodiment the oil is selected from the group consisting of medium-chain triglyceride, fish oil, olive oil and soybean oil.

In one embodiment the fatty acid is selected from a group selected from oleic acid, sodium acid, stearate and salts thereof.

In one embodiment the surfactant is selected from a group consisting of sorbitan esters such as, e.g., Span 20, Span 60 and Span 80, polysorbates such as, e.g., Polysorbate 20, Polysorbate 80 and Tween®, alkoxylated fatty acids, vegetable oil ethoxylates and ethoxylated fatty alcohols, such as, e.g., Brij® 35.

In one embodiment the ionizable lipid nanoparticle according to the various aspects further comprises at least one phenolic lipid. In one embodiment the ionizable lipid nanoparticle according to the various aspects further comprises at least one pSar lipid. In one embodiment the ionizable lipid nanoparticle according to the various aspects further comprises at least one polylactame. In one embodiment the ionizable lipid nanoparticle according to the various aspects further comprises at least one fatty alcohol. In one embodiment the ionizable lipid nanoparticle according to the various aspects further comprises at least one stearylated octaarginine. In one embodiment the ionizable lipid nanoparticle according to the various aspects further comprises at least one preservative. In one embodiment the ionizable lipid nanoparticle according to the various aspects further comprises at least one biocide.

In one embodiment the phenolic lipid is selected from a group consisting of anacardic acid, cardol and cardanol. In one embodiment the preservative is selected from a group consisting of benzyl alcohol, potassium sorbate, sodium benzoate, benzoic acid, phenoxyethanol.

In a fifth aspect, there is provided a method of producing the ionizable lipid nanoparticles according to the fourth aspect and the embodiments related thereto,
wherein the method comprises the following steps, in sequence:

a) providing a lipid phase comprising a compound according to any of claims 1 to 5, an ionizable lipid according to claim 6 or 7 or a product obtained according to claim 8,

b) providing an aqueous phase, and

c) mixing the lipid phase provided in a) with the aqueous phase provided in b) preferably wherein a microfluidic technique is used, and

d) obtaining a lipid nanoparticle.

In one embodiment the mixing can be carried out by a microfluidic technique. The microfluidic technique is based on both, turbulent and laminar flow. The lipid phase and the aqueous phase are introduced into microfluidic channels where they meet and mix. A microfluidic channel may have several geometries to provide a specific mixing efficiency, size control and scalability. In one embodiment the microfluidic channel can be selected from the group consisting of linear channel, multi-channel, "Y"-junction and "T"-junction and chaotic mixer. In one embodiment the mixing can occur under laminar flow to promote gentle and uniform mixing. In another embodiment the mixing can occur under turbulent flow which induces rapid mixing.

In another embodiment the mixing can be carried out by an ethanol injection method. This method provides a rapid injection of a lipid-containing ethanol solution into an aqueous phase to facilitate the self-assembly of nanoparticles.

In yet another embodiment the mixing can be carried out by combining the lipid phase with the aqueous phase under controlled conditions to achieve a nanoparticle formation.

Downstream processes may include desalting, buffer exchange, concentration methods, centrifugal ultrafiltration and diafiltration, wherein spin columns and filters may be used. Further, there may be carried out tangential flow filtration, size exclusion chromatography, ion exchange chromatography and/or sterile filtration, and optionally freezing and/or lyophilisation.

In a sixth aspect, there is provided a composition comprising the compound or the ionizable lipid or the ionizable lipid nanoparticle according to the various aspects.

In a preferred embodiment the composition is a pharmaceutical composition. In another embodiment, the composition is a cosmetic composition. In yet another embodiment the composition is a nutritional composition. In a further embodiment the composition is an agricultural composition.

A "pharmaceutical composition" as used herein refers to a composition, preparation or product comprising at least one main ingredient or substance and, optionally, at least one carrier. It may be used as medicine, medical device, adjuvant and health care product. It can further be used to prevent or treat disorders and diseases, to improve health condition, to influence physiological functions or to enable medical diagnosis.

A "cosmetic composition" as used herein refers to a composition, preparation or product comprising at least one ingredient, wherein the ingredient or the whole formulation is/are used to change the external appearance or smell of the human or animal body in line with socially determined and/or individual ideals. It can further be used for body care and penetrating and permeating skin barriers.

A "nutritional composition" as used herein refers to a composition, preparation or product in the field of nutrition. Non limiting examples of nutritional compositions are a nutrition supplement product, a nutritional substitute product and a main nutritional product. The nutrition may be food in solid, semi-solid, liquid or gaseous food. The application form of the nutrition may be oral, parenteral, sublingual, buccal, subcutaneous, intramuscular, intravenous or inhalative. A nutritional composition according to the invention may be selected from the group consisting of a beverage, drink, meat-based food, vegetarian food, vegan food, artificial food, genetically modified food, bar, mush, emulsion, suspension solution, injection, infusion, aerosol, capsule, paste, gel, lozenge, granule, tablet, foam and spray.

An "agricultural composition" as used herein refers to a composition, preparation or product that can be used for application on and/or in the agricultural land, plants, grassland, arable land and livestock as well against a pathogen feeding on a plant, e.g. a pest and a parasite. Non limiting examples of agricultural compositions are a crop and and/or vegetable and/or fruit and/or ornamental protection product, a crop and and/or vegetable and/or fruit and/or ornamental growth product, a plant protection product, a plant growth product, a soil protection product, an animal feed, seeds, genetically modified plants, a herbicide and an insecticide.

In a seventh aspect, there is provided a use of the compound or a use of the ionizable lipid or a use of the ionizable lipid nanoparticle or a use of the composition according to the various aspects for the manufacture or preparation of a medicament for treatment and/or prevention of a disease or health condition as an active ingredient or as a cargo of an active ingredient.

In an eighth aspect, there is provided a use of the compound or a use of the ionizable lipid or a use of the ionizable lipid nanoparticle or a use of the composition according to the various aspects for the manufacture or preparation of a medicament for treatment and/or prevention of a disease or health condition as an active ingredient or as a cargo of an active ingredient,

wherein at least one disease selected from the group of cancer, inflammatory diseases, autoimmune diseases, destroyed tissue, psychological disorders, gastrointestinal diseases and respiratory diseases is/are treated and/or prevented.

In one embodiment, the compound or ionizable lipid or ionizable lipid nanoparticle or composition according to the various aspects may be used for recombinant protein production. In another embodiment, the compound or ionizable lipid or ionizable lipid nanoparticle or composition according to the various aspects may be used for immune modulating.

In a certain embodiment, the dosage form of a cosmetic composition or a pharmaceutical composition is selected from the group consisting of solid dosage forms such as tablets, sublingual tablets, melting tablets, dragees, capsules, soft capsules, hard capsules, gastroresistant capsules, granules, powders and printlets, semi-solid dosage forms such as ointments, creams, pastes and gels, liquid dosage forms such as suspensions, crystal suspensions, emulsions, solutions, drops, nasal drops, eye drops, ear drops, syrups, juices, injection solutions, injection dispersions, injection solutions and injection solution concentrates, and further dosage forms such as suppositories, lozenges, effervescent tablets, effervescent powders, inhalation powders, plasters, metered dose inhalers, foams, sprays, sublingual sprays, shampoos, mouthwashes, vaginal suppositories, vaginal tablets and vaginal capsules.

In a certain embodiment, the application form of a cosmetic composition or a pharmaceutical composition is selected from the group consisting of aural, buccal, enteral, inhalative, intra-arterial (i.a.), intra-articular, intrabronchial, intraductal, intragluteal, intracardiac, intracavernous, intralesional, intralumbar, intralymphatic, intramammary, intramuscular (i.m.), intranasal, intraneural, intraocularintraosseous (i.o.), intraperitoneal (i.p.), intrapleural, intrapulmonary, intrathecal, intratracheal, intraurethral, intrauterine, intravenous, intraventricular, intravitreal, conjunctival, oral, parenteral, peroral, perineural, rectal, retrobulbar, subcutaneous (s.c.), sublingual, transdermal and vaginal.

Surprisingly it was found that the compounds being suitable for ionizable lipids along with the resulting ionizable LNPs according to the invention show high efficiency of molecule delivery to specific locations in target organisms. Fig. 1 & 2 show the efficient drug delivery in mice, wherein iLNPs according to the invention were used. Given that the invented products are able to pass through biomembranes, skin barrierers and even the blood-brain barrier, they can be used to deliver drugs precisely to a human target region or in cosmetic products. They are therefore just as suitable for delivering molecules to versatile target organisms like plant cells or organisms in the nutritional or agricultural sector.

In a ninth aspect, there is provided a use of the compound or the ionizable lipid or the ionizable lipid nanoparticle or the composition according to the various aspects as a cargo in a cosmetic product, in a nutritional product, in a diagnostic product, for recombinant protein production, or in an agricultural product.

Further aspects and advantages of the invention result from the following description of preferred embodiments and from the drawings along with the description of drawings according to the invention.

## Examples

Cytotoxicity assay

Cytotoxicity analysis of SYV ionizable lipids was performed by the XTT cell viability assay.

The cytotoxic effects of lipids were evaluated in vitro by XTT cytotoxic assay. Hep-G2 cells were seeded in a 96-well plate at 30 000 cells/100 μL and incubated for 24 hours. The medium was removed, cells were washed with PBS twice and the medium containing SYV ionizable lipids in concentrations: 10, 20, 40, 60, 100, 200, 400, 600, 800 and 1000 μM was added. Reference cells were prepared (non-treated) in wells adding fresh medium with 1% DMSO and blank (wells without cells). After 24 and 48-hours incubation, the XTT was added to each well. And the cells were incubated for 3 hours. The absorbance was measured at wavelength 450 nm and reference wavelength 660 nm in the microplate reader. Cell viability data were expressed as experiments' means ± standard deviation (SD). All experiments were performed in triplicates. The specific absorbance was calculated as follows:

Abs = Abs450nm(test) - Abs450nm(blank) - Abs660nm(test)

and the relative cell viability (%) was compared to control cells and calculated using the following equation:

$$Cell\ viability\ (\%\ of\ sample) = \frac{Abs\ sample}{Abs\ control} \times 100$$

The IC50 (half maximal inhibitory concentration) was calculated using ED50 Excel tool.

See **Fig. 12, Fig. 13, Fig. 14** and **Fig. 4.**

In-vivo biodistribution of mRNA-LNPs

LNPs were prepared by nanoprecipitation method using Spark or Ignite (Precision Nanosystems Inc.). The obtained LNP suspensions were diluted 10-fold in PBS 150 mM or Tris 60 mM. LNP purification, buffer exchange, and formulation concentration were performed using MWCO 30000 filters (Merck). Particle size and polydispersity of the LNPs were measured using Zetasizer (Malvern) before and after purification. The mRNA encapsulation efficiency was determined by the Quant-iT Ribogreen RNA assay.

An experiment was conducted involving acclimatized mice (6-8 week-old female BALB/c mice) that were administered dispersion of provided test formulations. During the acclimatization period and throughout the experiment, the mice were housed in IVC cages (3 mice per cage) under standard environmental conditions: temperature 22 °C ± 2 °C, relative humidity 55% ± 10%, and a 12-hour light/dark cycle. The animals had free access to autoclaved rodent feed (Altromin 1324) and were provided with autoclaved water ad libitum.

To investigate the accumulation and decay of bioluminescence associated with the administered mRNA-LNP formulations (v=100μL), the test formulations were injected intravenously/into the tail vein of mice randomly divided into groups of 3 mice (in triplicate).

For imaging purposes, the mice were administered luciferase substrate, specifically luciferin, in the form of a potassium salt solution dissolved in PBS (15 mg/mL), into the dorsal fold. Depending on their body weight, the mice received volumes of this solution corresponding to the recommended dose of 150 mg/kg body weight, i.e., 3 mg of luciferin per 20 g mouse. Each mouse was weighed, and the luciferin doses were individually adjusted.

Approximately 5 minutes after luciferin administration and just before the start of imaging, the mice were anesthetized using 4% isoflurane in an induction chamber. Subsequently, after placing the mice in the IVIS Spectrum CT chamber on the imaging platform and maintaining anesthesia with 2% isoflurane delivered via a nose cone, bioluminescence imaging was performed over a 15-20 minute time period. During signal acquisition, bioluminescence signal parameters were recorded. For Group 1, a 1-second acquisition was chosen at 4 hours and a 2-second acquisition at 24 hours; for the other groups, 2 and 5-second acquisitions were selected, respectively. At longer acquisition times (e.g., 1 minute, 15 seconds), the signal was oversaturated. Bioluminescence values were quantified by measuring photon flux (photons/second) in the region of interest (i.e., the entire mouse excluding the tail) from which the bioluminescence signal emanated, using Living IMAGE software (Caliper).

Bioluminescence imaging of the mice, reflecting the translational efficiency of the mRNA complexed with the carrier, was performed at two time points (24 and 48 hours post-intravenous injection of the provided formulations).

See **Fig. 1, Fig. 2** and **Fig. 3.**

Jurkat Cells transfection mRNA encoding luciferase

The aim was the evaluation of translational efficiency of lipofectamine Messenger Max and SyVento formulations on Jurkat cells model (T-cells) using Trilink Flue mRNA encoding luciferase.

Jurkat cells were seeded on a 96-well plate in the number 20 000 cells/well on the white opaque plate for luminescence readouts. The day before the seeding day, a Trilink mRNA mix with lipofectamine Messenger Reagent and Opti-MEM was prepared.

After 24 hours 5μl of mRNA-lipofectamine mix was gently pipetted and added for wells with cells to the following final concentrations: 500 ng/mL (50 ng mRNA per well), 200 ng/mL (20 ng mRNA per well), 100 ng/mL and (10 ng mRNA per well), then incubated for 24 hours in 37°C and 5% $CO_2$.

Three formulations (SYV1_LG_1_230924, SYV2_LP_1_230924, SYV3_LO_1_230924) were diluted in PBS and added to cells in the following concentrations: 500 ng/mL (50 ng mRNA per well), 200 ng/mL (20 ng mRNA per well), 100 ng/mL and (10 ng mRNA per well), then incubated for 24 hours in 37°C and 5% $CO_2$.

As a control formulation with reference lipid *SM-102* was prepared and added to cells in final concentration: 1000 ng/mL (100 ng mRNA per well), 500 ng/mL (500 ng mRNA per well), 200 ng/mL (20 ng mRNA per well), 100 ng/mL (10 ng mRNA per well), 10 ng/mL (1 ng mRNA per well), then incubated for 24 hours in 37°C and 5% $CO_2$.

The translational efficiency for plates was measured using the Bright-Glo™ Luciferase Assay (Promega) 24 hours after transfection - 105 μl of equilibrated to room temperature reagent was added to each well and then mixed gently. After 3 minutes luminescence was read using VarioScan Lux plate reader.

See **Fig. 5, Fig. 6** and **Fig. 7.**

Assessment of target silencing efficiency

Table 1

| **LNPs** | **Average particle size [nm]** | **Average gene silencing [0.35-2.80 μg/mL]** | **Maximal gene silencing (concentration [μg/mL])]** |
|---|---|---|---|
| **SYV1_MC3** | 65.00 | 68% | 75% [2.8] |
| **SYV2_LF_1+6** | 74.90 | 67% | 83% [2.8] |
| **SYV3_LF8** | 71.30 | 86% | 91% [1.4] |
| **SYV4_LO_1+6** | 61.40 | 84% | 93% [1.4] |
| **SYV5_LP_8** | 80.70 | 81% | 89% [0.7] |
| **SYV12_C12** | 103.6 | 72% | 80% [1.4] |

Surprisingly it was found that Lipid F, Lipid O and Lipid P exceed *D-Lin-MC3-DMA* and *C12-200* in the capacity of gene silencing.

Expression analysis of immune response-related factors

No significant changes in OAS1 expression were observed for the formulations comprising LNPs according to

the invention.

See **Fig. 11.**

Target silencing effectiveness

Formulations comprising LNPs according to the invention loaded with siRNA lead to a surprisingly effective target silencing.

See **Fig. 8, Fig. 9** and **Fig. 10.**

## Claims

1. A compound being represented by formula (A) or formula (B) or a salt or isomer thereof, preferably being suitable as lipid, particularly preferably being used as ionizable lipid

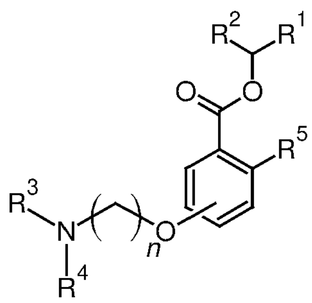

formula (A)

wherein $R_1$ is an alkyl, an alkenyl or an alkinyl,
wherein $R_2$ is selected from the group consisting of H, COOH, benzyl, phenol, an alkyl, and an alkenyl,
wherein $R_3$ and $R_4$ are independently selected from the group consisting of H, $NH_2$, benzyl, phenol, benzoic acid, an alkyl, and an alkenyl,
wherein $R_5$ is selected from the group consisting of H, $NH_2$, an alkyl, an alkenyl or an alkinyl,
wherein n is 1, 2, 3, 4, 5 or 6, preferably 1, 2, 3 or 4, more preferably 2 or 3, particularly preferably 2,

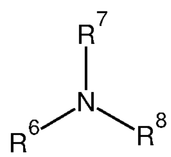

formula (B),

wherein $R_6$ is represented by formula (B-1)

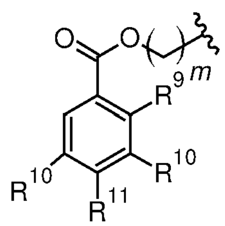

formula (B-1),

or a salt or isomer thereof,

wherein $R_7$ is represented by formula (B-1) or a salt or isomer thereof, or is selected from the group consisting of H, an alkyl with a total number of carbon atoms in the range from 1 to 7, a methanol group, an ethanol group, a propanol group, a butanol group or a pentanol group, preferably a methyl group,
wherein $R_8$ is represented by formula (B-1) or a salt or isomer thereof, or is selected from the group consisting of H, an alkyl with a total number of carbon atoms in the range from 1 to 7, a methanol group, an ethanol group, a propanol group, a butanol group or a pentanol group, preferably a methyl group or a propanol group,
wherein $R_9$ is selected from the group consisting of H, O-$R_{B1}$ or an alkyl,
wherein $R_{B1}$ is selected from the group consisting of H, OH or an alkyl,
wherein $R_{10}$ is selected from the group consisting of H, O-$R_{B2}$ or an alkyl,
wherein $R_{B2}$ is selected from the group consisting of H, OH or an alkyl
wherein $R_{11}$ is selected from the group consisting of H, O-$R_{B3}$ or an alkyl,
wherein $R_{B3}$ is selected from the group consisting of H, OH or an alkyl, and/or
wherein m is 1, 2, 3, 4, 5 or 6, preferably 2, 3, 4 or 5, particularly preferably 2 or 5,
preferably wherein at least one of $R_9$, $R_{10}$ or $R_{11}$ is selected from the group consisting of O-$R_{B1}$, O-$R_{B2}$ or O-$R_{B3}$.

2. The compound according to claim 1, wherein the at least one tertiary amine is selected from the group of a tertiary aliphatic amine, a tertiary cyclic amine and a tertiary aromatic amine, preferably wherein the at least one tertiary amine is a tertiary aliphatic amine.

3. The compound according to claim 1 or 2, wherein at least one of $R_1$, $R_2$, $R_5$, $R_9$, $R_{10}$, $R_{11}$, $R_{B1}$, $R_{B2}$ and $R_{B3}$ is/are an alkyl, an alkenyl or an alkinyl with a total number of carbon atoms of 1, 2, 3, 4, 5, 6, 7, 8, 9, 10, 11, 12, 13, 14, 15, 16, 17, 18, 19, 20, 21, 22, 23, 24, 25, or above, preferably with a total number of carbon atoms in the range from 6 to 24, more preferably wherein at least one of $R_1$, $R_2$, $R_5$, $R_9$, $R_{10}$, $R_{11}$, $R_{B1}$, $R_{B2}$ and $R_{B3}$ has a total number of carbon atoms of 10, 12, 13, 15, 16, 17 or 18.

4. The compound according to claim 3, wherein the alkyl or alkenyl or alkinyl comprises one or more hetero atoms as chemical residue, preferably wherein the hetero atom(s) is/are oxygen atom(s).

5. The compound according to any of the preceding claims, wherein the compound is represented by formula (1), (2), (3), (4), (5), (6), (7), (8), (9), (10), (11), (12), (13) or (14) or a salt or isomer thereof, preferably being suitable as lipid, particularly preferably being used as ionizable lipid

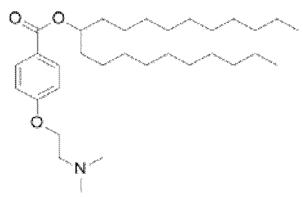

formula (1)

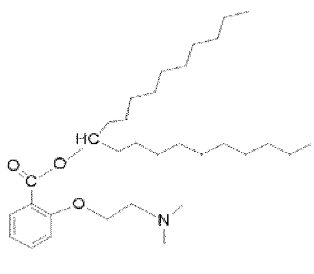

formula (2)

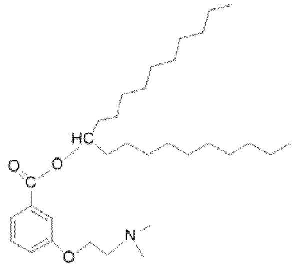

formula (3)

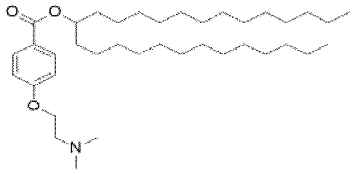

formula (4)

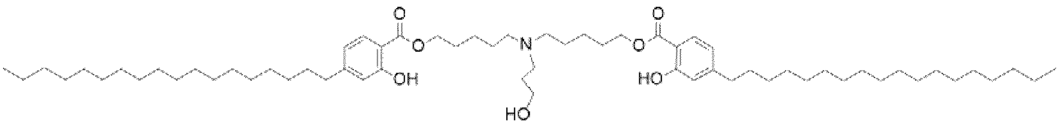

formula (5)

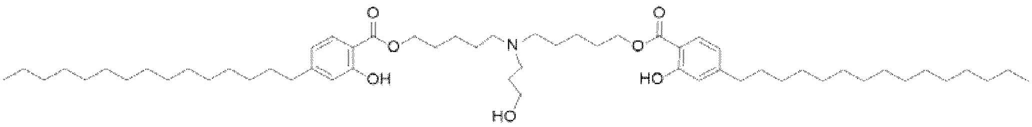

formula (6)

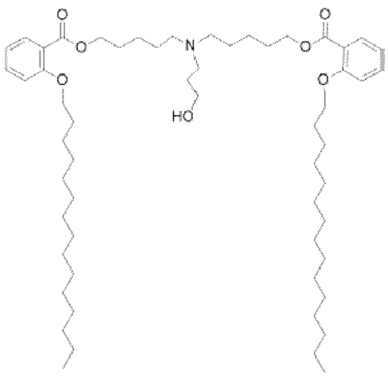

formula (7)

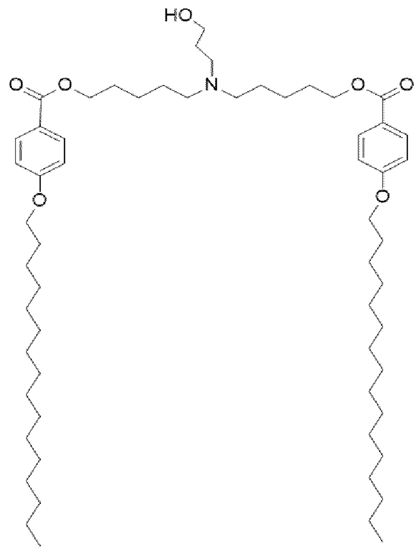

formula (8)

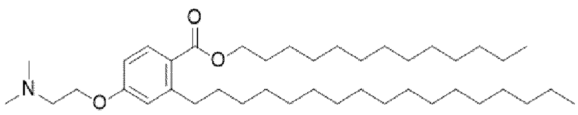

formula (9)

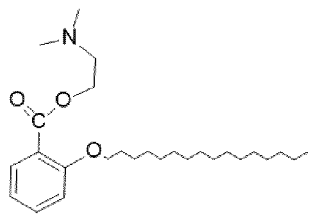

formula (10)

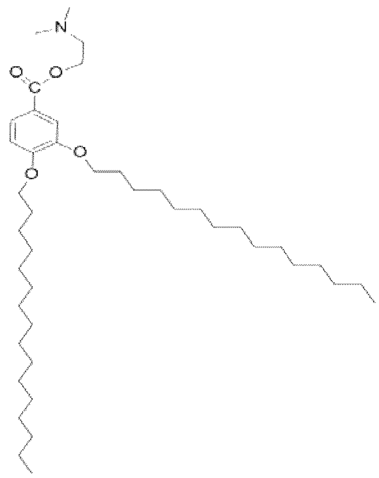

formula (11)

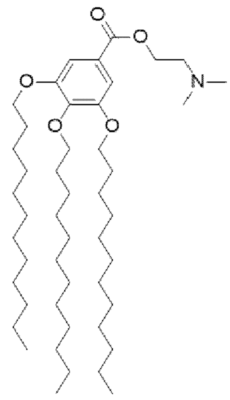

formula (12)

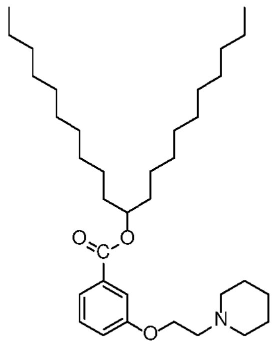

formula (13)

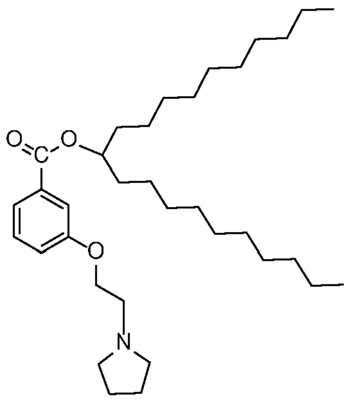

formula (14).

6. An ionizable lipid comprising a hydroxybenzoic acid structure, preferably wherein the ionizable lipid has a chemical structure of formula (A) or (B), wherein formula (B) includes formula (B-1), as defined in claim 1, preferably wherein one or two hydroxybenzoic acid(s) is/are present.

7. The ionizable lipid according to claim 6, wherein at least one tertiary amine is present and wherein the at least one tertiary amine has at least one chemical residue, wherein the chemical residue is selected from the group consisting of H, an alkyl, a methanol group, an ethanol group, a propanol group, a butanol group or a pentanol group, preferably a methyl group or a propanol group,

preferably wherein the tertiary amine is connected to the phenolic oxygen atom of the hydroxybenzoic acid via an alkyl group,
preferably wherein the tertiary amine is connected to the oxygen atom of the carbonyl group of the hydroxybenzoic acid via an alkyl group,
preferably wherein the carboxyl group of the hydroxybenzoic acid is connected to the tertiary amine via an alkyl group,
preferably wherein the aromatic group of the hydroxybenzoic acid has H, an ether or an alkyl as a chemical residue,
preferably wherein the tail group as chemical residue of the ether and/or the aromatic group of the hydroxybenzoic acid and/or the oxygen atom of the carboxyl group is an alkyl, an alkenyl or an alkinyl with a total number of carbon atoms of 1, 2, 3, 4, 5, 6, 7, 8, 9, 10, 11, 12, 13, 14, 15, 16, 17, 18, 19, 20, 21, 22, 23, 24, 25, or above, more preferably with a total number of carbon atoms in the range from 6 to 24, particularly preferably with a total number of carbon atoms of 10, 12, 13, 15, 16, 17 or 18.

**8.** A method of producing the compound according to any of claims 1 to 5, or the lipid according to claim 6 or 7, wherein the method comprises the following steps, in sequence:

a) providing a raw material comprising a structure of hydroxbenzoic acid or a derivative or a salt thereof,
b) mixing the provided raw material with a substance for elongating the tail group of the provided raw material,
c) adding a substance to the mixture obtained in b) for hydrating a derivative of a hydroxyl group of the mixture obtained in b),
d) adding a substance comprising a tertiary amine to the mixture obtained in c), and
e) obtaining a product comprising a hydroxybenzoic acid structure connected to a tertiary amine and further comprising a lipophilic tail group.

**9.** An ionizable lipid nanoparticle comprising the compound according to any of claims 1 to 5 or the lipid according to claim 6 or 7.

**10.** An ionizable lipid nanoparticle according to claim 9, further comprising

structure lipids, wherein the structure lipid is phospholipid and/or sphingolipid, preferably wherein the phospholipid is selected from the group consisting of PC, PE, PA, PG, PI, PS and LPL, preferably wherein the sphingolipid is sphingomyelin, more preferably wherein the phospholipid is selected from the group of DSPC, DOPE, HSPC, DPPC, DSPG, DSPS and DPPA,
sterols, preferably wherein the sterol is cholesterol and/or sitosterol,
lipids modified with hydrophilic polymers, preferably wherein the lipid modified with hydrophilic polymers is PEGylated lipid, more preferably wherein the PEGylated lipid is DMG-PEG and/or DSPE-PEG and/or DSG-PEG, and optionally
SORT lipids, preferably wherein the SORT lipid comprises DODAP, DOTAP, DPDAB, DODMA 14PA, 18BMP, 18PA, DC-cholesterol and/or 5A2-SC8,
and optionally
antioxidants and/or oils and/or fatty acids and/or fatty acid salts and/or surfactants.

**11.** An method of producing the ionizable lipid nanoparticles according to claim 9 or 10,
wherein the method comprises the following steps, in sequence:

a) providing a lipid phase comprising a compound according to any of claims 1 to 5, an ionizable lipid according to claim 6 or 7 or a product obtained according to claim 8,
b) providing an aqueous phase,
c) mixing the lipid phase provided in a) with the aqueous phase provided in b), preferably wherein a microfluidic technique is used, and
d) obtaining a lipid nanoparticle.

**12.** A composition comprising the compound according to any of claims 1 to 5, the ionizable lipid according to claim 6 or 7 or the ionizable lipid nanoparticle according to claim 9 or 10,
preferably wherein the composition is a pharmaceutical composition, a cosmetic composition, a nutritional composition or an agricultural composition.

**13.** A compound according to any of claims 1 to 5, or an ionizable lipid according to claim 6 or 7, or an ionizable lipid nanoparticle according to claim 9 or 10, or a composition according to claim 12 for use in a method of treatment and/or prevention as an active ingredient or as a cargo of an active ingredient.

**14.** A compound according to any of claims 1 to 5, or an ionizable lipid according to claim 6 or 7, or an ionizable lipid nanoparticle according to claim 9 or 10, or a composition according to claim 12 for use in a method of treatment and/or prevention as an active ingredient or as a cargo of an active ingredient,
wherein at least one disease selected from the group of cancer, inflammatory diseases, autoimmune diseases, destroyed tissue, psychological disorders, gastrointestinal diseases and respiratory diseases is/are treated and/or prevented.

**15.** A use of the compound according to any of claims 1 to 5, or a use of the lipid according to claim 6 or 7, or a use of the ionizable lipid nanoparticle according to claim 9 or 10, or a use of the composition according to claim 12 as a cargo in a cosmetic product, in a nutritional product, in a diagnostic product, for recombinant protein production, or in an agricultural product.

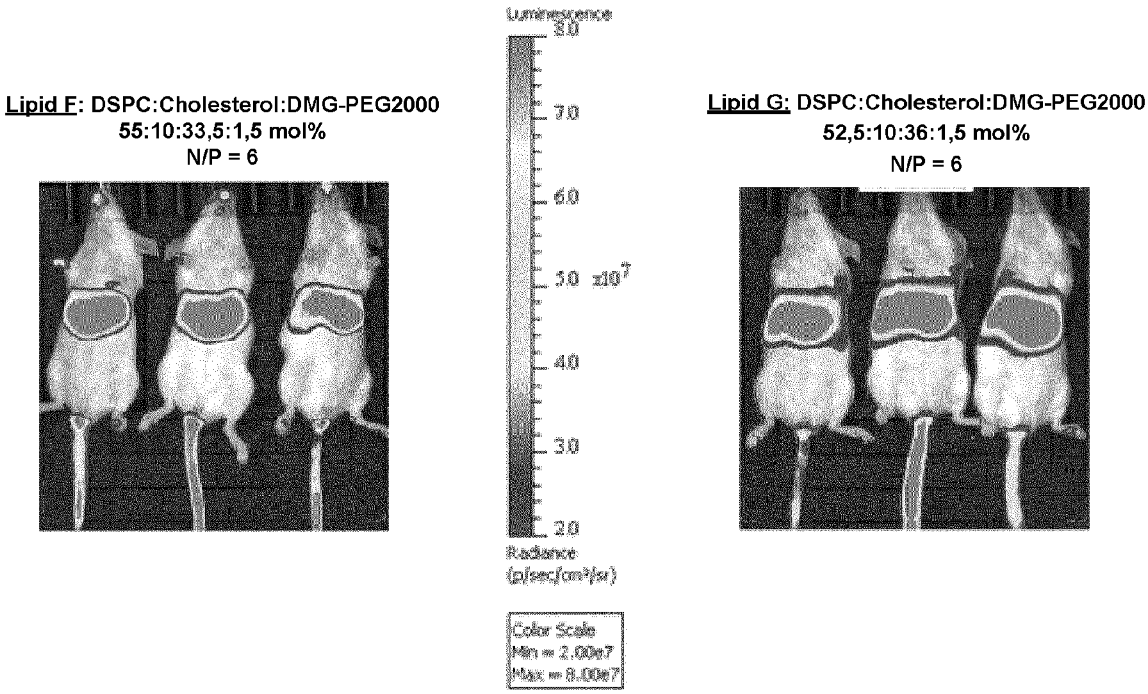
Lipid F: DSPC:Cholesterol:DMG-PEG2000
55:10:33,5:1,5 mol%
N/P = 6
Luminescence
8.0
7.0
6.0
5.0 x10^7
4.0
3.0
2.0
Radiance
(p/sec/cm²/sr)
Color Scale
Min = 2.00e7
Max = 8.00e7
Lipid G: DSPC:Cholesterol:DMG-PEG2000
52,5:10:36:1,5 mol%
N/P = 6

Fig. 1

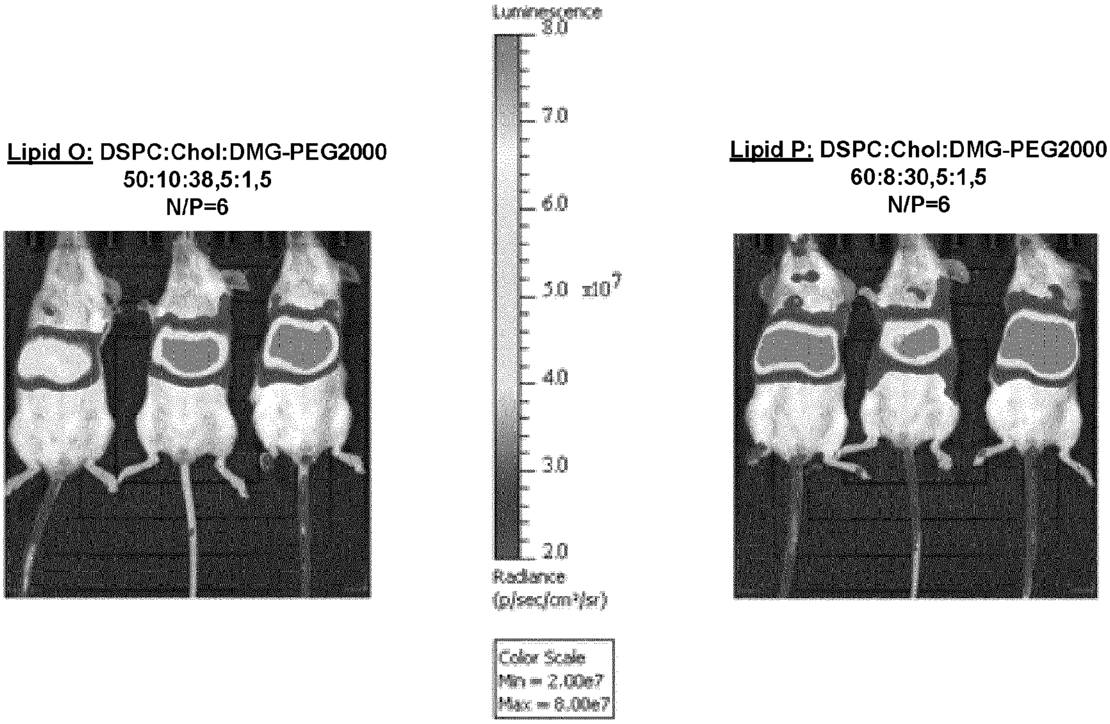
Lipid O: DSPC:Chol:DMG-PEG2000
50:10:38,5:1,5
N/P=6
Luminescence
8.0
7.0
6.0
5.0 x10^7
4.0
3.0
2.0
Radiance
(p/sec/cm²/sr)
Color Scale
Min = 2.00e7
Max = 8.00e7
Lipid P: DSPC:Chol:DMG-PEG2000
60:8:30,5:1,5
N/P=6

Fig. 2

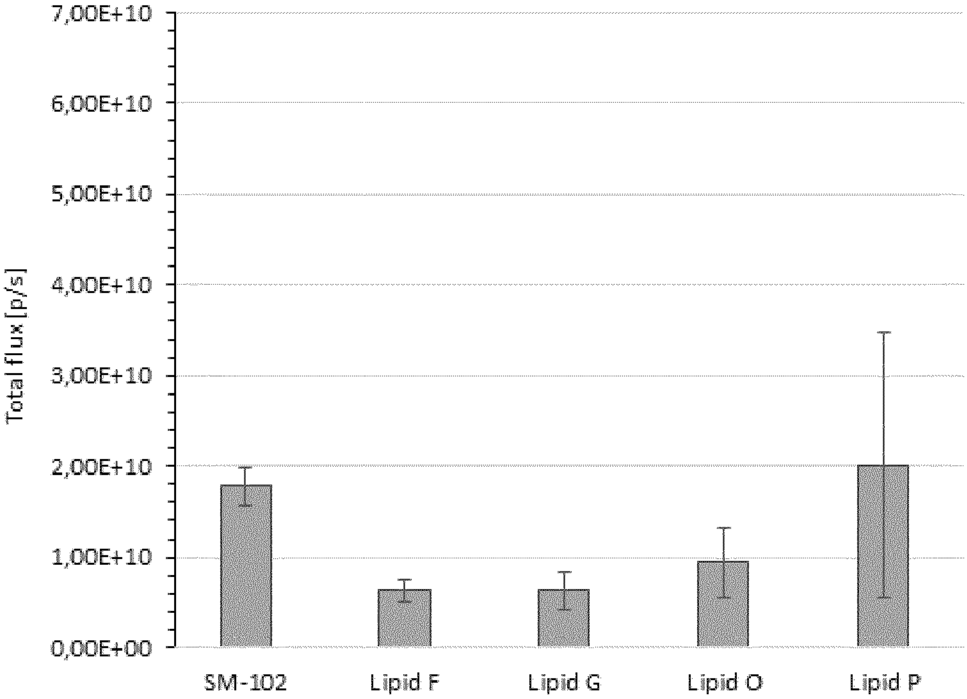
7,00E+10
6,00E+10
5,00E+10
4,00E+10
3,00E+10
2,00E+10
1,00E+10
0,00E+00
Total flux [p/s]
SM-102
Lipid F
Lipid G
Lipid O
Lipid P

Fig. 3

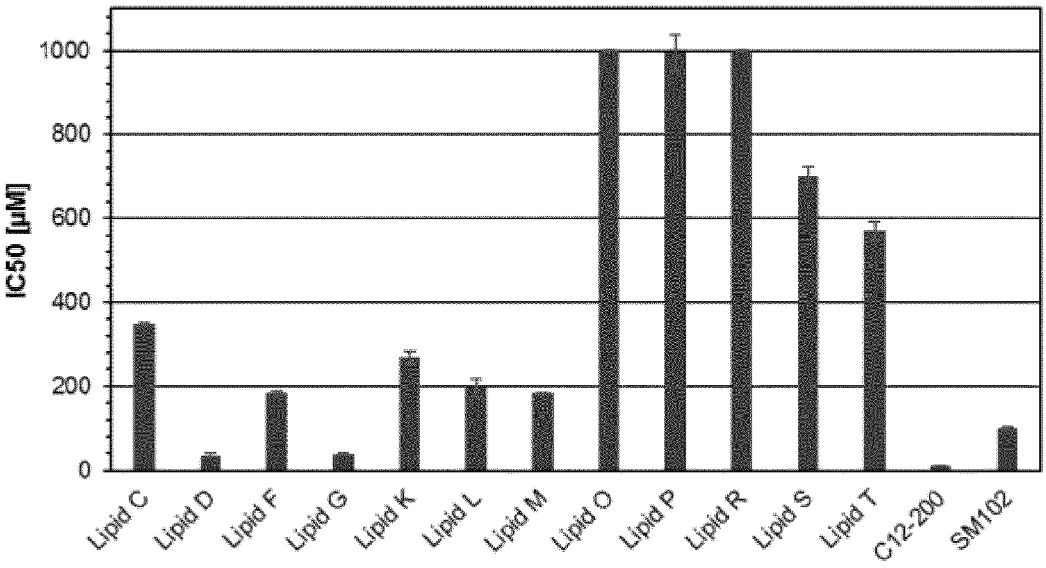


*IC50 Values (Half Maximal Inhibitory Concentration) of Ionizable Lipids [The higher the value, the better]*

Fig. 4

mRNA TRANSFECTION EFFICIENCY
PROTEIN SYNTHESIS LEVEL (LUCIFERASE)

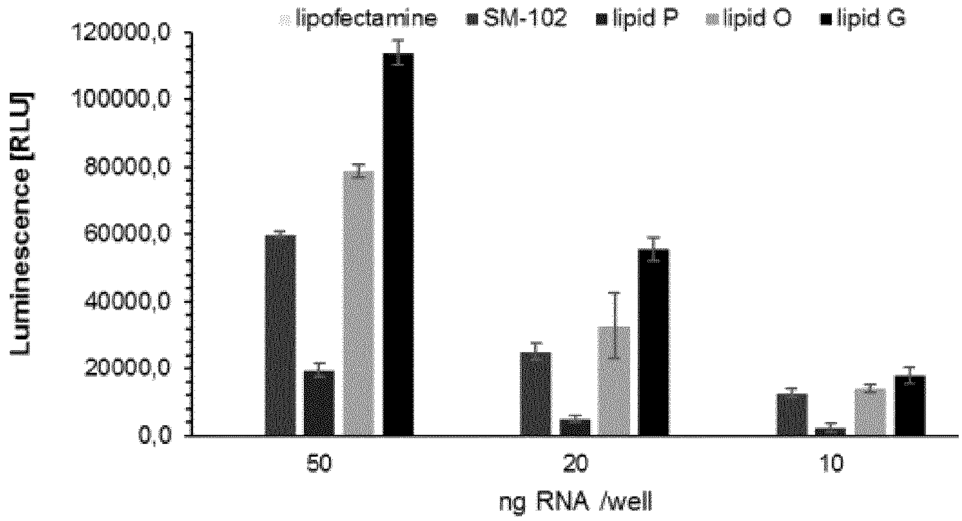


*Luminescence of Jurkat Cells (Immortalized T Lymphocytes) 24 Hours After Transfection Using LNP and Lipofectamine Formulations at 50 ng, 20 ng, and 10 ng mRNA per Well*

Fig. 5

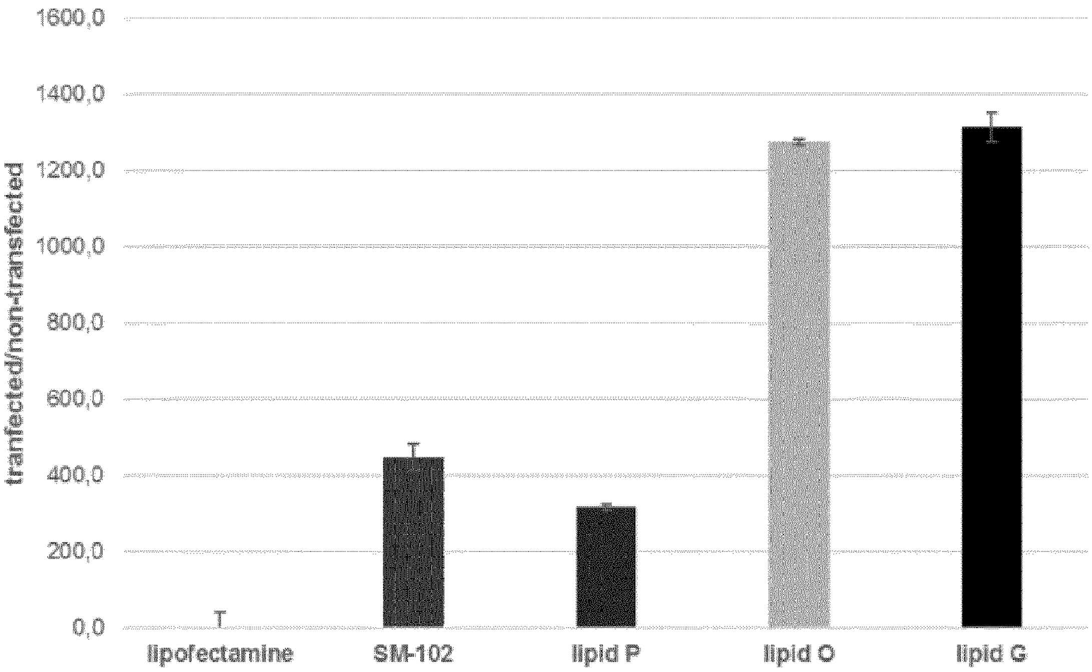
1600,0
1400,0
1200,0
1000,0
800,0
600,0
400,0
200,0
0,0
tranfected/non-transfected
lipofectamine
SM-102
lipid P
lipid O
lipid G

Fig. 6

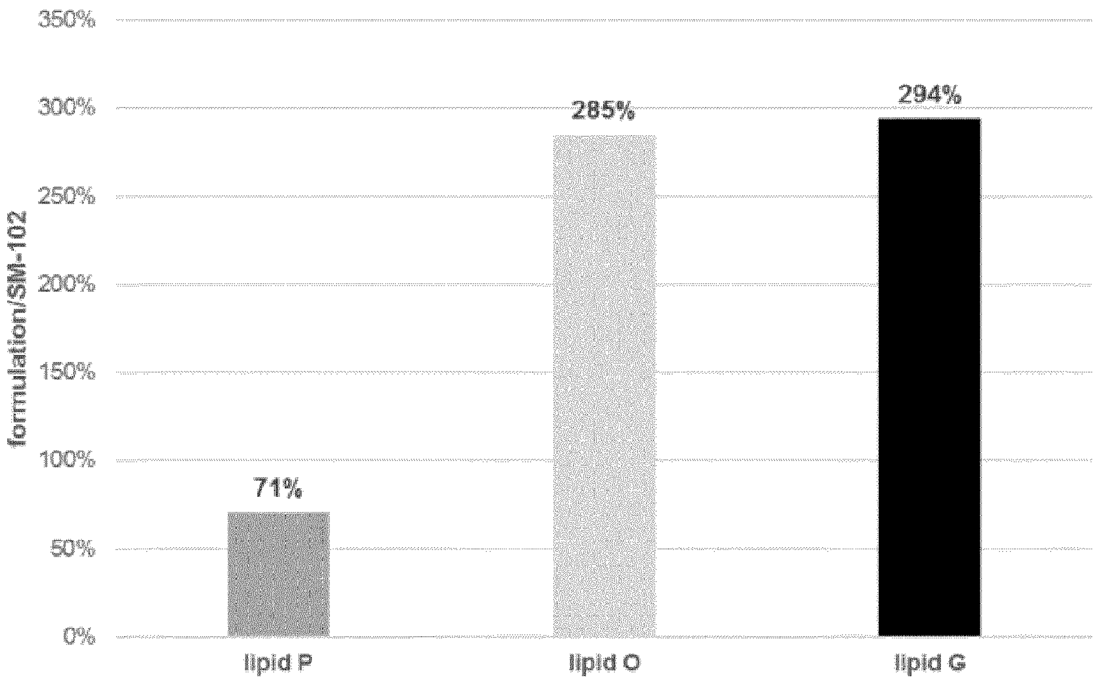
350%
300%
250%
200%
150%
100%
50%
0%
formulation/SM-102
71%
285%
294%
lipid P
lipid O
lipid G

Fig. 7

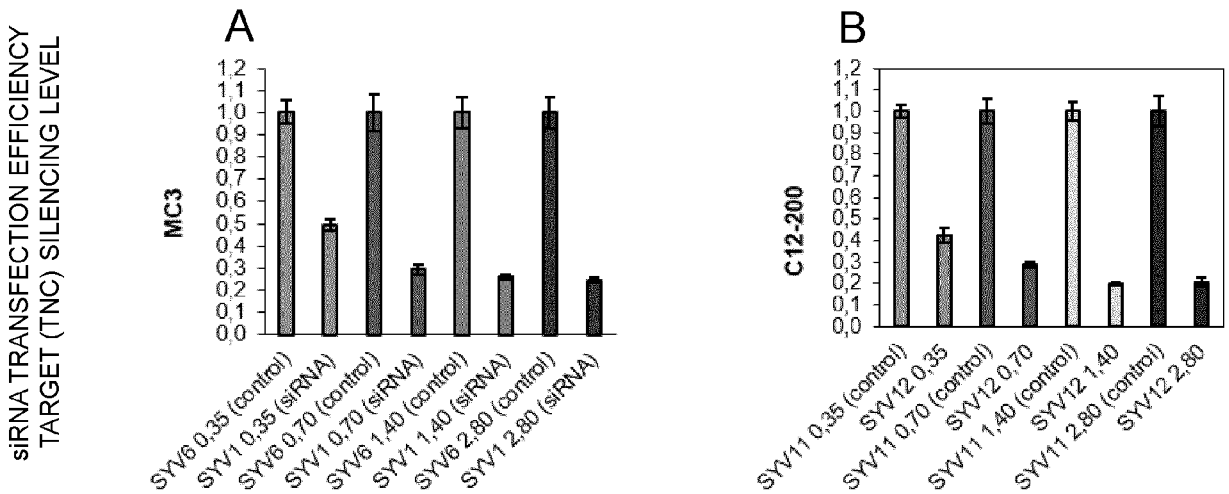
siRNA TRANSFECTION EFFICIENCY
TARGET (TNC) SILENCING LEVEL
A
MC3
1,2
1,1
1,0
0,9
0,8
0,7
0,6
0,5
0,4
0,3
0,2
0,1
0,0
SYV6 0,35 (control)
SYV1 0,35 (siRNA)
SYV6 0,70 (control)
SYV1 0,70 (siRNA)
SYV6 1,40 (control)
SYV1 1,40 (siRNA)
SYV6 2,80 (control)
SYV1 2,80 (siRNA)
B
C12-200
SYV11 0,35 (control)
SYV12 0,35
SYV11 0,70 (control)
SYV12 0,70
SYV11 1,40 (control)
SYV12 1,40
SYV11 2,80 (control)
SYV12 2,80

Fig. 8

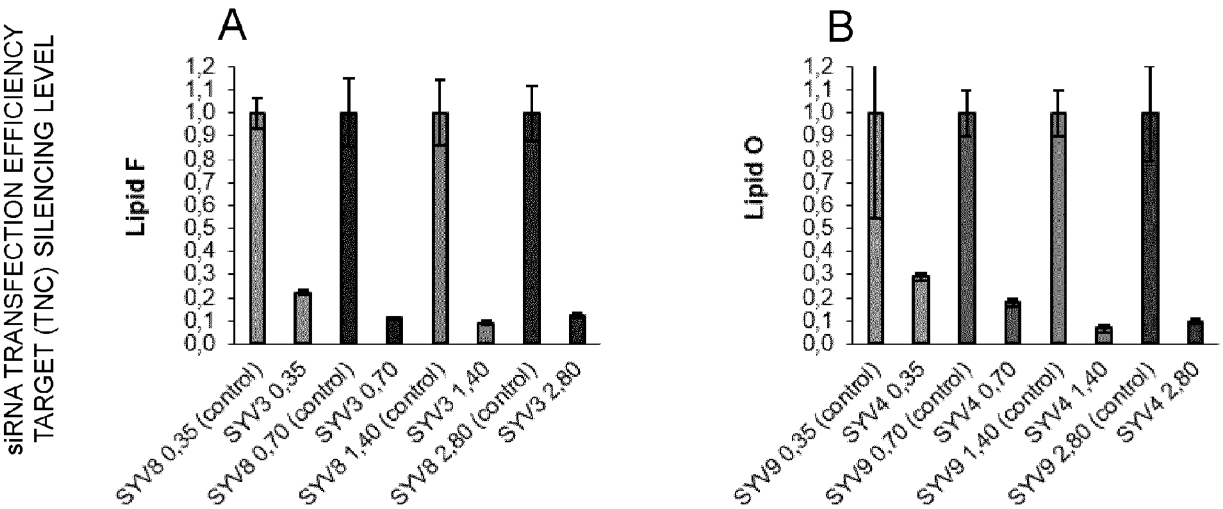
siRNA TRANSFECTION EFFICIENCY
TARGET (TNC) SILENCING LEVEL
A
Lipid F
1,2
1,1
1,0
0,9
0,8
0,7
0,6
0,5
0,4
0,3
0,2
0,1
0,0
SYV8 0,35 (control)
SYV3 0,35
SYV8 0,70 (control)
SYV3 0,70
SYV8 1,40 (control)
SYV3 1,40
SYV8 2,80 (control)
SYV3 2,80
B
Lipid O
SYV9 0,35 (control)
SYV4 0,35
SYV9 0,70 (control)
SYV4 0,70
SYV9 1,40 (control)
SYV4 1,40
SYV9 2,80 (control)
SYV4 2,80

Fig. 9

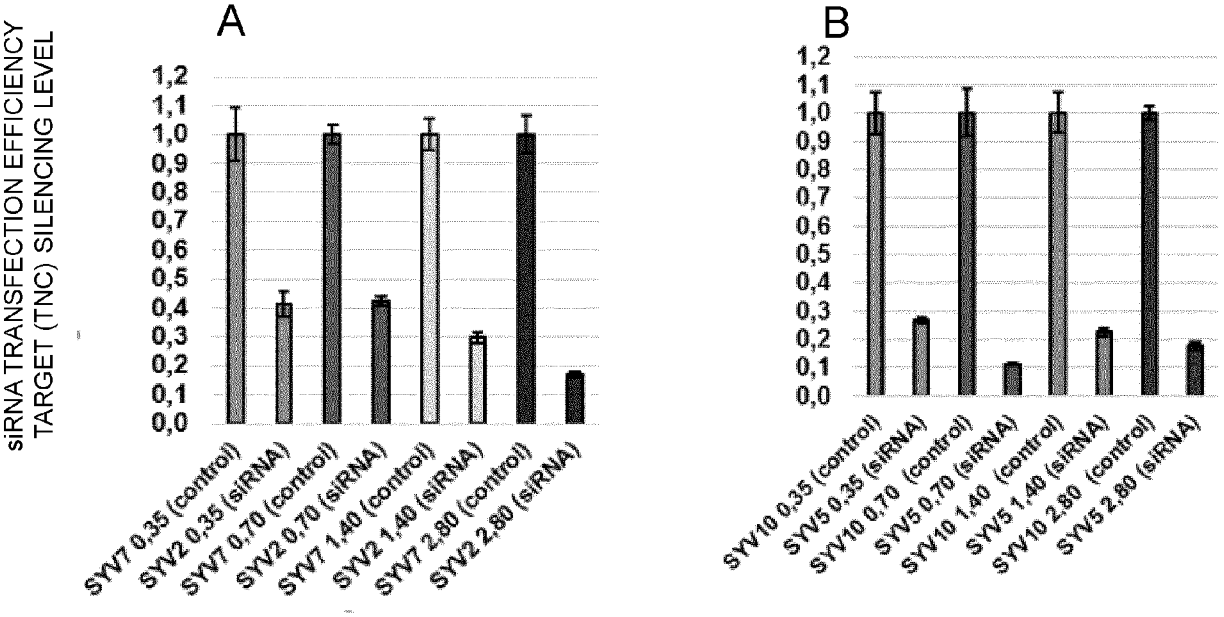

A
B
siRNA TRANSFECTION EFFICIENCY
TARGET (TNC) SILENCING LEVEL
1,2
1,1
1,0
0,9
0,8
0,7
0,6
0,5
0,4
0,3
0,2
0,1
0,0
SYV7 0,35 (control)
SYV2 0,35 (siRNA)
SYV7 0,70 (control)
SYV2 0,70 (siRNA)
SYV7 1,40 (control)
SYV2 1,40 (siRNA)
SYV7 2,80 (control)
SYV2 2,80 (siRNA)
SYV10 0,35 (control)
SYV5 0,35 (siRNA)
SYV10 0,70 (control)
SYV5 0,70 (siRNA)
SYV10 1,40 (control)
SYV5 1,40 (siRNA)
SYV10 2,80 (control)
SYV5 2,80 (siRNA)


Fig. 10

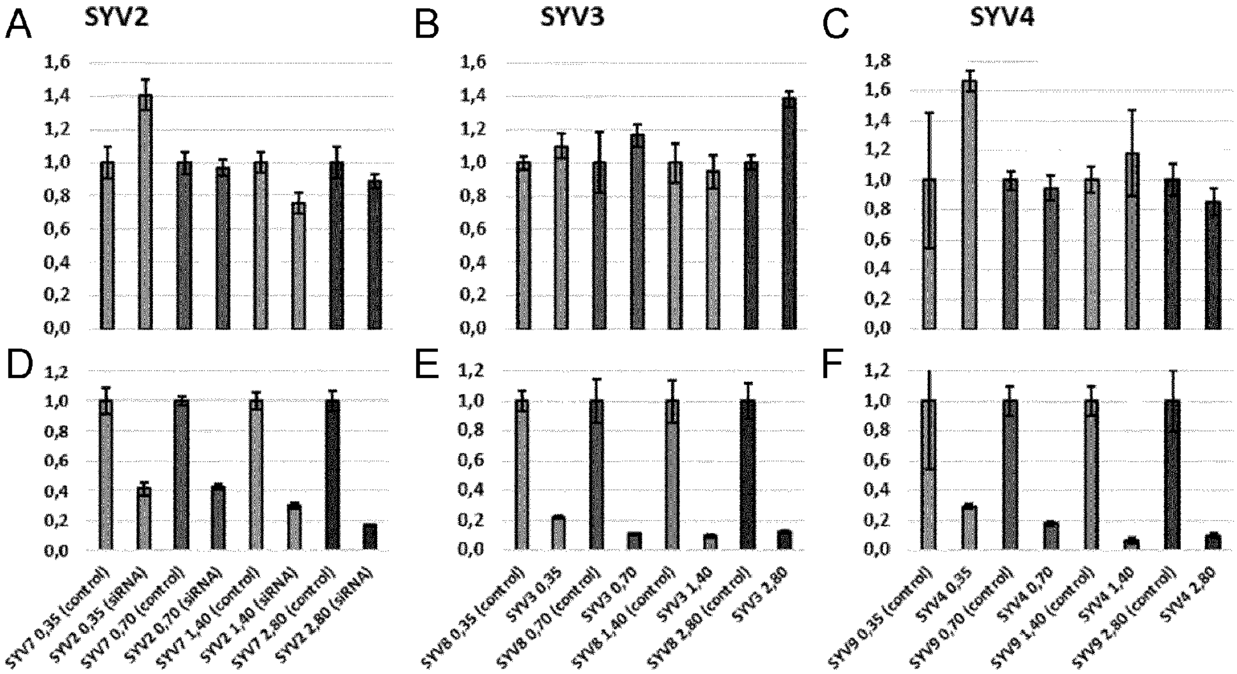
A
SYV2
B
SYV3
C
SYV4
D
E
F
SYV7 0,35 (control)
SYV2 0,35 (siRNA)
SYV7 0,70 (control)
SYV2 0,70 (siRNA)
SYV7 1,40 (control)
SYV2 1,40 (siRNA)
SYV7 2,80 (control)
SYV2 2,80 (siRNA)
SYV8 0,35 (control)
SYV3 0,35
SYV8 0,70 (control)
SYV3 0,70
SYV8 1,40 (control)
SYV3 1,40
SYV8 2,80 (control)
SYV3 2,80
SYV9 0,35 (control)
SYV4 0,35
SYV9 0,70 (control)
SYV4 0,70
SYV9 1,40 (control)
SYV4 1,40
SYV9 2,80 (control)
SYV4 2,80

Fig. 11

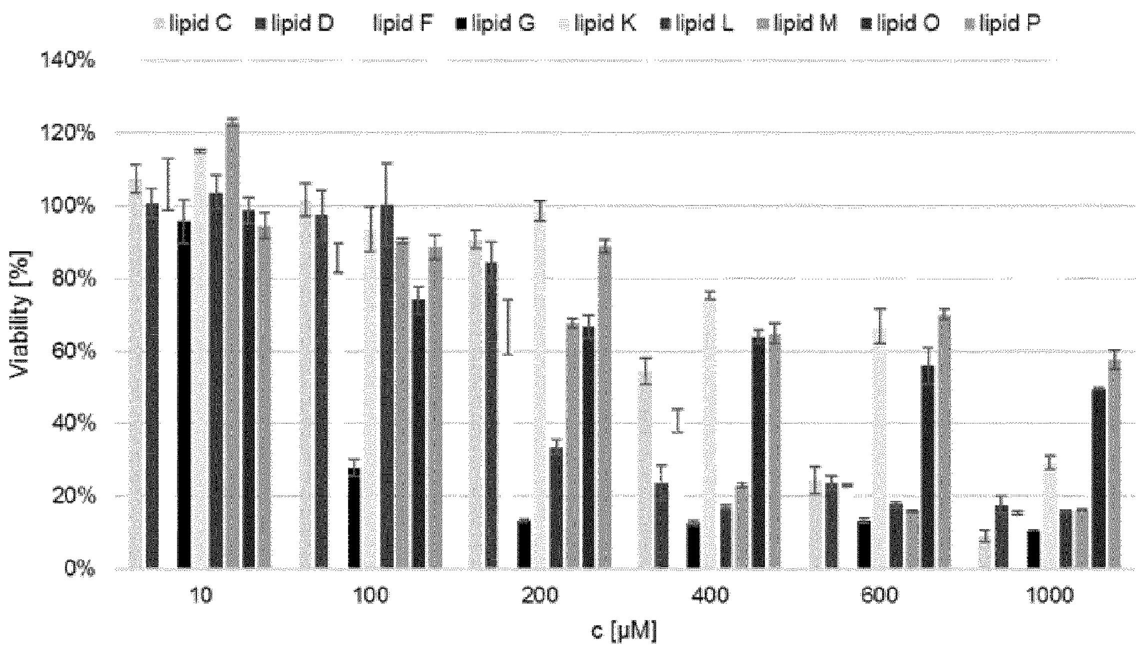
lipid C
lipid D
lipid F
lipid G
lipid K
lipid L
lipid M
lipid O
lipid P
140%
120%
100%
80%
60%
40%
20%
0%
Viability [%]
10
100
200
400
600
1000
c [µM]

Fig. 12

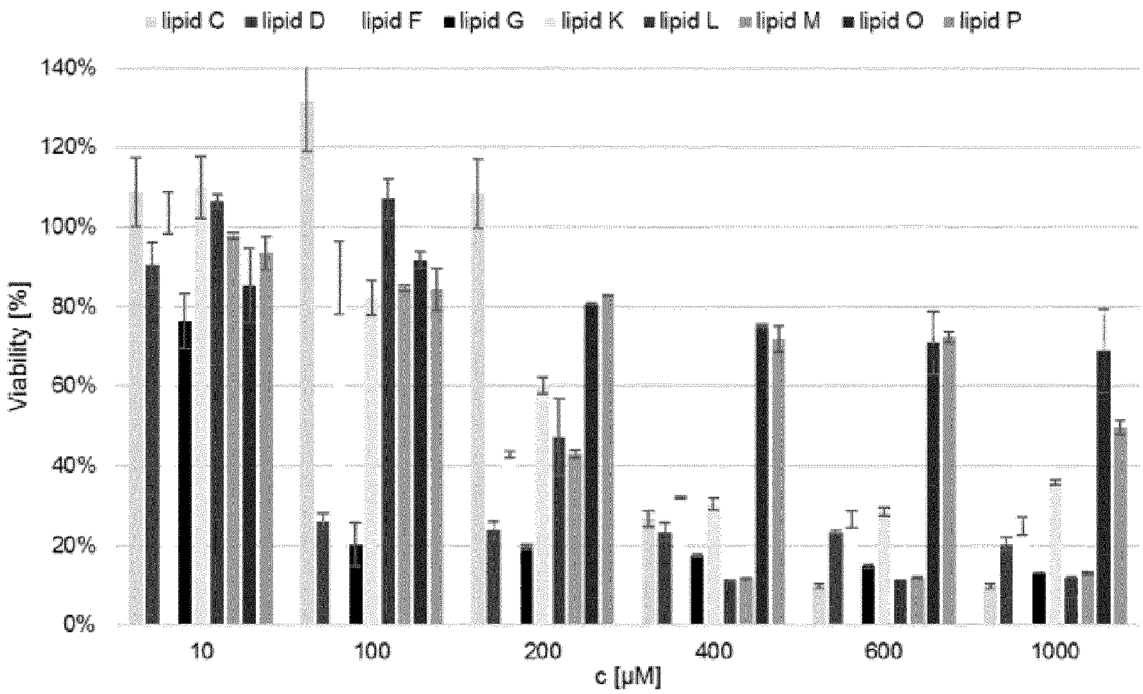
lipid C
lipid D
lipid F
lipid G
lipid K
lipid L
lipid M
lipid O
lipid P
140%
120%
100%
80%
60%
40%
20%
0%
Viability [%]
10
100
200
400
600
1000
c [µM]

Fig. 13

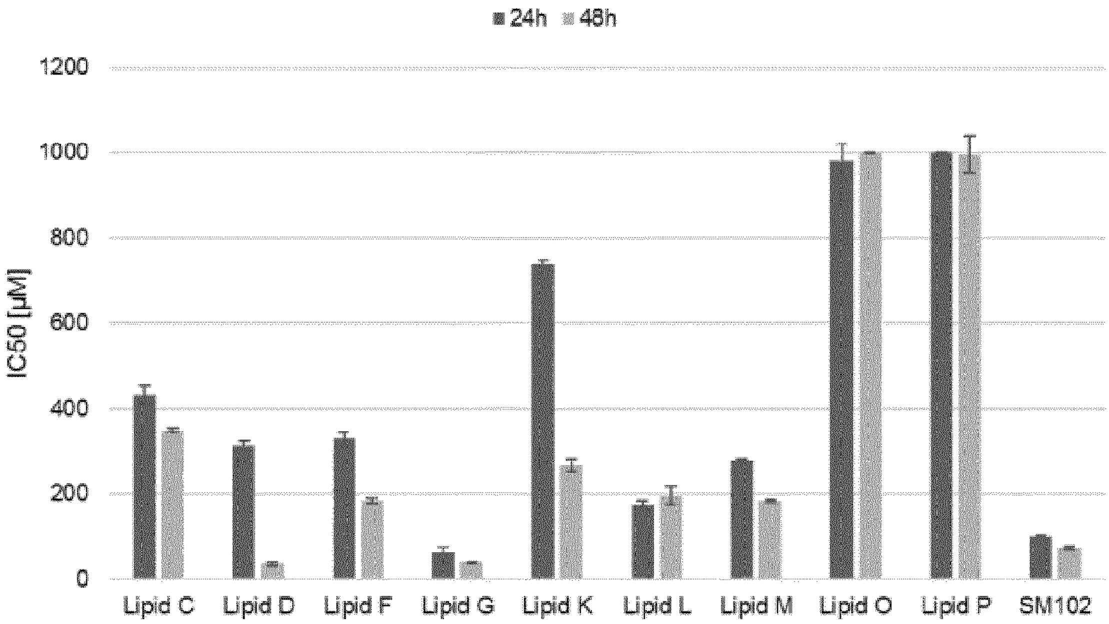
24h
48h
1200
1000
800
600
400
200
0
IC50 [µM]
Lipid C
Lipid D
Lipid F
Lipid G
Lipid K
Lipid L
Lipid M
Lipid O
Lipid P
SM102

Fig. 14

Europäisches Patentamt
European Patent Office
Office européen des brevets

# EUROPEAN SEARCH REPORT

Application Number
EP 25 15 1378

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 115 286 523 A (INST OF BASIC MEDICAL AND ONCOLOGY CHINESE ACADEMY OF SCIENCES) 4 November 2022 (2022-11-04) * paragraphs [0043] - [0045] * * paragraphs [0116] - [0117] * * examples 44-46 * * paragraphs [0002], [0003] * ----- | 1-15 | INV. C07C217/16 C07C219/14 C07D295/088 A61K9/1272 |
| X | WO 2024/223953 A1 (QUANTOOM BIOSCIENCES S A [BE]; UNIV BRUXELLES [BE]) 31 October 2024 (2024-10-31) * page 157, lines 13-20; compounds CX2, CX3 * * examples 3-7 * ----- | 1-15 | |
| X | SAHOO DIPANKAR ET AL: "The Constitutional Isomerism of One-Component Ionizable Amphiphilic Janus Dendrimers Orchestrates the Total and Targeted Activities of mRNA Delivery", JOURNAL OF THE AMERICAN CHEMICAL SOCIETY, vol. 146, no. 6, 2 February 2024 (2024-02-02), pages 3627-3634, XP093288778, ISSN: 0002-7863, DOI: 10.1021/jacs.3c13569 Retrieved from the Internet: URL:https://pubs.acs.org/doi/pdf/10.1021/jacs.3c13569> * figure 1 * * abstract * ----- | 1-15 | TECHNICAL FIELDS SEARCHED (IPC) C07C C07D A61K |
| X | JP H08 325154 A (MITSUI TOATSU CHEMICALS) 10 December 1996 (1996-12-10) * examples 7, 39 * * paragraph [0006] * ----- -/-- | 1-3,5-8, 12-14 | |

1

The present search report has been drawn up for all claims

| Place of search | Date of completion of the search | Examiner |
|---|---|---|
| The Hague | 23 June 2025 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 25 15 1378

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 716 644 A (ALBERS H ET AL) 13 February 1973 (1973-02-13) * examples 7, 8, 25, 30 * * abstract * | 1-3,6-8, 12-14 | |
| X | US 4 503 244 A (MOELLER HINRICH [DE] ET AL) 5 March 1985 (1985-03-05) * claims 1, 10 * | 1-3,6,7, 12-15 | |
| X | STEBANI U ET AL: "UNCONVENTIONAL MESOGENS OF HYPERBRANCHED AMIDES AND CORRESPONDING AMMONIUM DERIVATIVES", ADVANCED MATERIALS, VCH PUBLISHERS, DE, vol. 7, no. 6, 1 June 1995 (1995-06-01), pages 578-581, XP000508456, ISSN: 0935-9648, DOI: 10.1002/ADMA.19950070617 * page 579; compounds 4a, 4b, 5a, 5b * | 1-3,6-12 | |
| X | WINSTEAD MELDRUM B ET AL: "Dialkylaminoethyl Esters of Some Alkoxybenzoic Acids", JOURNAL OF THE AMERICAN CHEMICAL SOCIETY, vol. 77, no. 3, 1955, pages 772-774, XP093288888, * page 773, table 1 * * page 773, right-hand column, lines 17-28 * | 1-4,6,7, 12-14 | TECHNICAL FIELDS SEARCHED (IPC) |

The present search report has been drawn up for all claims

1

| Place of search | Date of completion of the search | Examiner |
|---|---|---|
| The Hague | 23 June 2025 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 25 15 1378

This annex lists the patent family members relating to the patent documents cited in the above-mentioned European search report. The members are as contained in the European Patent Office EDP file on
The European Patent Office is in no way liable for these particulars which are merely given for the purpose of information.

23-06-2025

| Patent document cited in search report | Publication date | Patent family member(s) | Publication date |
|---|---|---|---|
| CN 115286523 A | 04-11-2022 | CN 115286523 A | 04-11-2022 |
| | | CN 117486738 A | 02-02-2024 |
| | | CN 117843506 A | 09-04-2024 |
| WO 2024223953 A1 | 31-10-2024 | NONE | |
| JP H08325154 A | 10-12-1996 | NONE | |
| US 3716644 A | 13-02-1973 | NONE | |
| US 4503244 A | 05-03-1985 | AT E25927 T1 | 15-04-1987 |
| | | DE 3301313 A1 | 19-07-1984 |
| | | EP 0114051 A1 | 25-07-1984 |
| | | JP H0452245 B2 | 21-08-1992 |
| | | JP S59137448 A | 07-08-1984 |
| | | US 4503244 A | 05-03-1985 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 11766408 B2
- WO 2021055833 A1


### Non-patent literature cited in the description


- **TANG et al.** *Ionizable Lipid Nanoparticles for mRNA Delivery*, 2023
- *CHEMICAL ABSTRACTS*, 1857341-90-2